# EUROPEAN PATENT APPLICATION

(11) **EP 2 292 266 A1**
(43) Date of publication of application: **09.03.2011**
(21) Application number: 09168877.0
(22) Date of filing: 27.08.2009
(51) Int. Cl.: A61K 39/395

(54) **Treating cancer by modulating copine III**

(71) Applicant: Novartis Forschungsstiftung, Zweigniederlassung, 4058 Basel (CH)
(72) Inventor: Heinrich, Constanze, 73733 Esslingen (DE); Hofsteenge, Jan, 4153 Reinach (CH); Hynes, Nancy, 4059 Basel (CH); Keller, Claudia, 4133 Pratteln (CH)
(74) Representative: Favre, Nicolas

(57) **Abstract**

The present invention relates to a method for treating cancer in a subject by modulating copine III, by administering to said subject a therapeutically effective amount of a modulator of copine III.

SEQUENCE LISTING

<110> Novartisforschungsstiftung, Zweigniederlassung Friedrich
      Miescher Institute for Biomedical Research

<120> Treating cancer by modulating copine III

<130> 53761

<160> 8

<170> PatentIn version 3.3

<210> 1
<211> 19
<212> PRT
<213> Homo sapiens

<400> 1

Ser Gly Ser Gly Pro Thr Ala Glu Asn Pro Glu Tyr Leu Gly Leu Asp
1               5                   10                  15


Val Pro Val



<210> 2
<211> 19
<212> RNA
<213> Mus musculus

<400> 2
ccaguuaucu guuaaucaa                                                    19


<210> 3
<211> 19
<212> RNA
<213> Mus musculus

<400> 3
ggcaaucuuu ccaaaguaa                                                    19


<210> 4
<211> 19
<212> RNA
<213> Mus musculus

<400> 4
gcggcugccg gaauuuacc                                                    19


<210> 5
<211> 59

<212> DNA
<213> Mus musculus

<400> 5
ccgggcggct gccggaattt accttctcga gggtaaattc cggcagccgc tttttaatt 59


<210> 6
<211> 57
<212> DNA
<213> Mus musculus

<400> 6
ccggccctac tgcaatggaa tccaactcga gttggattcc attgcagtag ggttttt 57


<210> 7
<211> 57
<212> DNA
<213> Mus musculus

<400> 7
ccgggcacag aaaggattaa gaattctcga gaattcttaa tcctttctgt gcttttt 57


<210> 8
<211> 537
<212> PRT
<213> Homo sapiens

<400> 8

Met Ala Ala Gln Cys Val Thr Lys Val Ala Leu Asn Val Ser Cys Ala
1               5                   10                  15


Asn Leu Leu Asp Lys Asp Ile Gly Ser Lys Ser Asp Pro Leu Cys Val
            20                  25                  30


Leu Phe Leu Asn Thr Ser Gly Gln Gln Trp Tyr Glu Val Glu Arg Thr
        35                  40                  45


Glu Arg Ile Lys Asn Cys Leu Asn Pro Gln Phe Ser Lys Thr Phe Ile
    50                  55                  60


Ile Asp Tyr Tyr Phe Glu Val Val Gln Lys Leu Lys Phe Gly Val Tyr
65                  70                  75                  80


Asp Ile Asp Asn Lys Thr Ile Glu Leu Ser Asp Asp Asp Phe Leu Gly
                85                  90                  95


Glu Cys Glu Cys Thr Leu Gly Gln Ile Val Ser Ser Lys Lys Leu Thr

            100                 105                 110

Arg Pro Leu Val Met Lys Thr Gly Arg Pro Ala Gly Lys Gly Ser Ile
        115                 120                 125

Thr Ile Ser Ala Glu Glu Ile Lys Asp Asn Arg Val Val Leu Phe Glu
    130                 135                 140

Met Glu Ala Arg Lys Leu Asp Asn Lys Asp Leu Phe Gly Lys Ser Asp
145                 150                 155                 160

Pro Tyr Leu Glu Phe His Lys Gln Thr Ser Asp Gly Asn Trp Leu Met
                165                 170                 175

Val His Arg Thr Glu Val Val Lys Asn Asn Leu Asn Pro Val Trp Arg
            180                 185                 190

Pro Phe Lys Ile Ser Leu Asn Ser Leu Cys Tyr Gly Asp Met Asp Lys
        195                 200                 205

Thr Ile Lys Val Glu Cys Tyr Asp Tyr Asp Asn Asp Gly Ser His Asp
    210                 215                 220

Leu Ile Gly Thr Phe Gln Thr Thr Met Thr Lys Leu Lys Glu Ala Ser
225                 230                 235                 240

Arg Ser Ser Pro Val Glu Phe Glu Cys Ile Asn Glu Lys Lys Arg Gln
                245                 250                 255

Lys Lys Lys Ser Tyr Lys Asn Ser Gly Val Ile Ser Val Lys Gln Cys
            260                 265                 270

Glu Ile Thr Val Glu Cys Thr Phe Leu Asp Tyr Ile Met Gly Gly Cys
        275                 280                 285

Gln Leu Asn Phe Thr Val Gly Val Asp Phe Thr Gly Ser Asn Gly Asp
    290                 295                 300

Pro Arg Ser Pro Asp Ser Leu His Tyr Ile Ser Pro Asn Gly Val Asn
305                 310                 315                 320

Glu Tyr Leu Thr Ala Leu Trp Ser Val Gly Leu Val Ile Gln Asp Tyr
                325                 330                 335

Asp Ala Asp Lys Met Phe Pro Ala Phe Gly Phe Gly Ala Gln Ile Pro
            340                 345                 350

Pro Gln Trp Gln Val Ser His Glu Phe Pro Met Asn Phe Asn Pro Ser
        355                 360                 365

Asn Pro Tyr Cys Asn Gly Ile Gln Gly Ile Val Glu Ala Tyr Arg Ser
    370                 375                 380

Cys Leu Pro Gln Ile Lys Leu Tyr Gly Pro Thr Asn Phe Ser Pro Ile
385                 390                 395                 400

Ile Asn His Val Ala Arg Phe Ala Ala Ala Ala Thr Gln Gln Gln Thr
                405                 410                 415

Ala Ser Gln Tyr Phe Val Leu Leu Ile Ile Thr Asp Gly Val Ile Thr
            420                 425                 430

Asp Leu Asp Glu Thr Arg Gln Ala Ile Val Asn Ala Ser Arg Leu Pro
        435                 440                 445

Met Ser Ile Ile Ile Val Gly Val Gly Gly Ala Asp Phe Ser Ala Met
    450                 455                 460

Glu Phe Leu Asp Gly Asp Gly Gly Ser Leu Arg Ser Pro Leu Gly Glu
465                 470                 475                 480

Val Ala Ile Arg Asp Ile Val Gln Phe Val Pro Phe Arg Gln Phe Gln
                485                 490                 495

Asn Ala Pro Lys Glu Ala Leu Ala Gln Cys Val Leu Ala Glu Ile Pro
            500                 505                 510

Gln Gln Val Val Gly Tyr Phe Asn Thr Tyr Lys Leu Leu Pro Pro Lys
        515                 520                 525

Asn Pro Ala Thr Lys Gln Gln Lys Gln
    530                 535

## Description

### Field of the Invention

The present invention relates to a method of treating cancer by modulating copine III.

### Background of the Invention

Cancers and malignant tumors are characterized by continuous cell proliferation and cell death and are related causally to both genetics and the environment. Genes whose expression are associated with cancer, and the products of said genes, are of potentially great importance as cancer markers in the early diagnosis and prognosis of various cancers, as well as potential targets in cancer treatment. Cancer is the a leading cause of human death next to coronary disease. In the United States, cancer causes the death of over half-million people each year and about two million new cases of cancer are diagnosed each year.
The identification of new genes essential for the growth of tumors has been an objective of cancer research over the past several decades.

The ErbB2/Her-2/Neu receptor belongs to the type I receptor tyrosine kinase (RTK) family also including EGFR, ErbB3 and ErbB4 (Schlessinger, 2000). Alterations in EGFR or ErbB2 promoting constitutive receptor activation have been implicated in development of many human cancers. Patients whose tumors show ErbB receptor alterations tend to have an aggressive disease and poor prognosis (Slamon *et al.,* 1987; Holbro and Hynes, 2004; Hynes and Lane, 2005; Ross and Fletcher, 1998). Upon binding of EGF-related ligands to ErbBs, receptor homo- and heterodimers form, leading to kinase activation and phosphorylation of specific tyrosine residues in the cytoplasmic tail (Hazan *et al.,* 1990; Hynes and Lane, 2005). ErbB2 has no ligand, but is the favored dimerization partner of the other family members (Graus-Porta *et al.,* 1997). ErbB3, although able to bind ligands, has impaired kinase activity. ErbB2 is the preferred heterodimerization partner of ErbB3, thereby coupling ErbB2 to the PI3K pathway (Holbro *et al.,* 2003). ErbB2, via activation of signaling pathways including Erk and PI3K/Akt mediates numerous cellular processes important for tumorigenesis including proliferation, survival and migration, (Citri and Yarden, 2006; Keely *et al.,* 1997; Klemke *et al.,* 1997; Schlessinger, 2000; Yarden and Sliwkowski, 2001).
The five major tyrosine phosphorylation sites, Tyr1023/1028, Tyr1139/1144, Tyr1196/1201, Tyr1222/1227 and Tyr1248/1253 (human/rat) and their role in ErbB2 signaling has been studied by various means. Tyr1028 couples to a pathway implicated in negative regulation of NeuT transforming potential, whereas the four other sites have a potentiating effect. Tyr1144, Tyr1201 and Tyr1227 signal through the Ras/Erk pathway by binding Grb2, Crk and Shc (Akiyama *et al.,* 1991; Dankort *et al.,* 2001; Dankort *et al.,* 1997), which also binds Tyr1253 (Schulze *et al.,* 2005). The role of pTyr residues has also been examined in migration assays. In T47D breast tumor cells, Tyr1196 and Tyr1222 have each been linked to ErbB2-mediated cell migration via PLCγ and Memo, respectively (Marone *et al.,* 2004; Meira *et al.,* 2009)

In order to identify new genes essential for the growth of tumors and for the development of metastasis, the present inventors set out in the present study to identify novel binding proteins for Tyr1248 and to link them to an ErbB2-mediated phenotype. Using a pTyr1248 encompassing peptide in an affinity pull-down approach followed by relative quantification by mass spectrometry, the present inventors identified copine III (CPNE3), which belongs to a family of Ca²⁺-dependent phospholipid binding proteins first found in the ciliate protozoan *Paramecium tetraurelia.* Copines are conserved from plants to humans, where nine *CPNE* genes have been identified (*CPNE I - IX*) (Tomsig and Creutz, 2002). All copines possess two N-terminal C2 domains (C2Ds) followed by a von Willebrand Alike or A domain. C2Ds were originally identified in conventional protein kinase C isoforms and have been found in a growing number of eukaryotic proteins (Nalefski and Falke, 1996). The copine A domain, while similar to the extracellular A domain of integrins, is cytoplasmic and thought to mediate protein-protein interactions (Tomsig and Creutz, 2002).
Although widely expressed throughout the eukaryotes, indeed, most cells express multiple copines, relatively little is known about their specific roles. Moreover, although there had been reports that copine III might be up-regulated in tumor cells in response to ErbB2 overexpression (Gharbi et a/., 2002; White *et al.,* 2004; Duran *et al.,* 2008) those reports were contradictory in themselves and the results of these studies diverged from those of other published studies. The present inventors nevertheless examined the role of copine III downstream of ErbB2 signaling and show here that copine III is a Ca²⁺-dependent, phospholipid-binding protein that interacts with activated ErbB2 at the plasma membrane of tumor cells. Moreover, the present inventors identified the focal adhesion (FA) associated scaffold protein RACK1 as a copine III binding partner. Importantly, the present inventors also show that copine III is required for ErbB2-dependent cell migration.

### Summary of the invention

Hence, the present inventors have now surprisingly found that the copine III interacts with activated ErbB2 at the plasma membrane of tumor cells, and is required for ErbB2-dependent cell migration.

The present invention hence provides a method for treating cancer in a subject by modulating copine III via the administration of a therapeutically effective amount of a modulator of copine III to said subject. In some embodiments, copine III is modulated by an inhibitor, such as an antibody. Alternatively, the inhibitor decreases or silences the expression of copine III, and is for instance a siRNA. In some embodiments, the subject is a mammal, for instance a human subject.

The methods of the invention are suitable for all cancers dependent on the activity of copine III. In one embodiment, the ErbB2-pathway is misregulated in the cells of the cancer. Example of such a misregulation are amplifications or overexpression of the *ErbB2* gene. In one embodiment, the cancer is a cancer of the breast, an ovarian cancer, a stomach cancer, or a uterine cancer, such as uterine serous endometrial carcinoma, which cancers are generally associated with a misregulation of the ErbB2-pathway. In some embodiments, the cancer is treated by inhibiting/reducing metastasis formation.

The present invention also encompasses a siRNA decreasing or silencing the expression of copine III or an antibody specifically binding to copine III for use as a medicament to treat cancer. Alternatively, the antibody can inhibit the binding of copine III to its ligands.

The present invention also provides methods of screening for agents able to modulate the expression of copine III expression and/or biological activity, which method comprises: (i) contacting a copine III polypeptide or a fragment thereof having the biological activity of copine III, a polynucleotide encoding such a polypeptide or polypeptide fragment, an expression vector comprising such a polynucleotide or a cell comprising such an expression vector, and a test substance under conditions that in the absence of the test substance would permit copine III expression and/or biological activity; and (ii) determining the amount of copine III expression and/or biological activity, to determine whether the test substance modulates copine III biological activity and/or expression, wherein a test substance which modulates copine III biological activity and/or expression is a potential therapeutical agent to treat cancer.

In addition, the present invention also encompasses a method of diagnosing cancer comprising the step of assessing the level of expression of copine III in a sample from a subject.

These and other aspects of the present invention should be apparent to those skilled in the art, from the teachings herein.

### Brief Description of the Drawings

The following drawings are illustrative of embodiments of the invention and are not meant to limit the scope of the invention as encompassed by the claims.

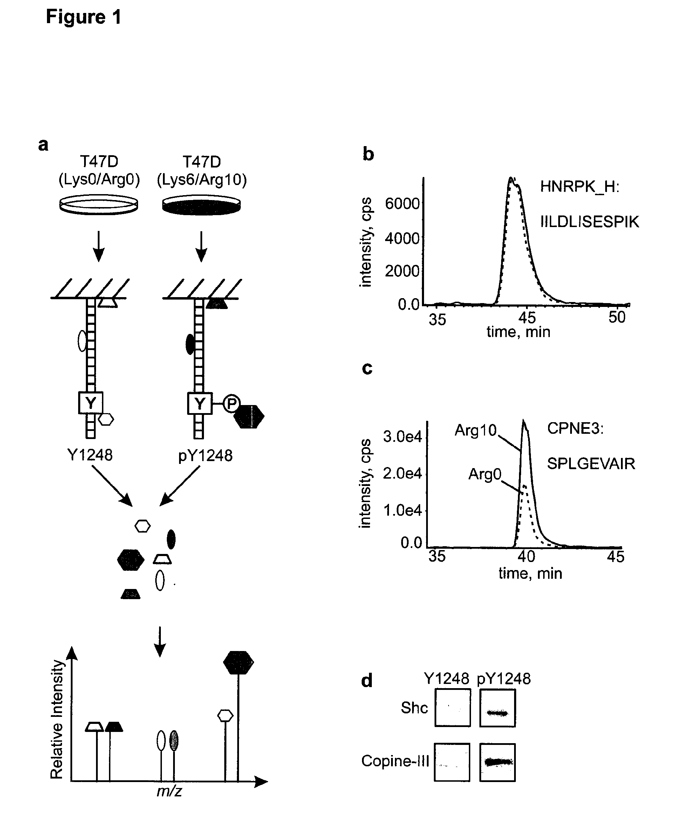

- **Figure 1:**: **Copine III preferentially binds to pTyr1248 of the ErbB2 receptor.** (**a**) Schematic illustration of the peptide affinity method used to identify proteins binding to the pTyr1248 of ErbB2. 15-mer peptides encompassing the Tyr1248 of ErbB2, either phosphorylated or not, were bound to magnetic beads and incubated with protein extract from isotopically lysine (Lys6) and arginine (Arg10) labeled T47D cells (SILAC).

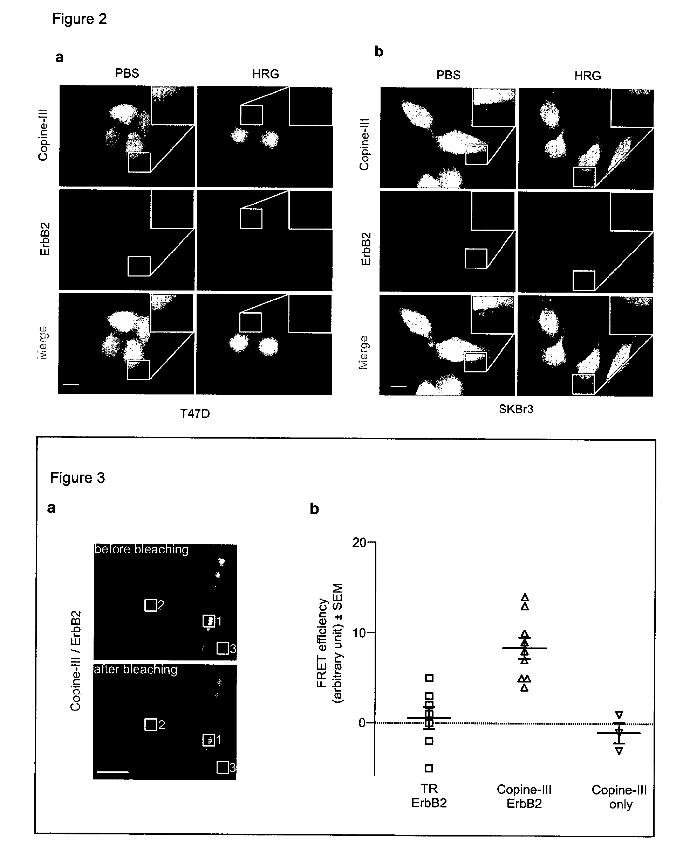

- **Figure 2:**: Identification and relative quantification of proteins was done by mass spectrometry. (b and c) Multiple reaction monitoring of single peptide pairs. One transition for each form of a peptide (Lys0/Arg0 and Lys6/Arg10) was measured over time. Peak areas were compared using Analyst 1.4.1. Representative spectra for a peptide pair from a background protein (Heterogeneous nuclear ribonucleoprotein K) and a specific binding protein (copine III) are shown. (d) Western blot analysis of Shc and copine III binding to Tyr/pTyr1248. Peptide pulldowns using HRG stimulated T47D cell extracts were performed and the bound proteins were monitored by western blot. **Co-localization of copine III and ErbB2 at the plasma membrane.** T47D (**a**) and SKBr3 (**b**) cells were starved overnight and stimulated with HRG for 20min, control cells were treated with PBS in parallel. Copine III and ErbB2 were visualized with their respective antibodies. The bottom panels are merged images, where yellow represents regions of co-localization of copine-III and ErbB2 (scale bar: 10µm). Inserts show 2x zooms of boxed regions showing the co-localization of copine III and ErbB2 at the plasma membrane.
- **Figure 3:**: **FRET acceptor photobleaching shows the interaction of copine III and ErbB2 at the plasma membrane.** SKBr3 cells were starved overnight and stimulated with HRG for 20min. Copine III and ErbB2 were labeled with their respective primary antibodies and visualized with an Alexa488-labeled anti-mouse and a Cy3-labeled anti-rabbit secondary antibody, respectively. (**a**) Representative images of a single cell before and after acceptor bleaching are shown. Three regions of interest (boxes 1-3) were taken for FRET efficiency calculations (scale bar: 5µm). (**b**) Quantification of the FRET acceptor photobleaching experiments (ErbB2/copine III, middle). In each experiment 4-10 individual cells were measured. As a control transferrin receptor (TR) was visualized with an Alexa488- and ErbB2 with Cy3-labeled secondary antibody and FRET efficiency was measured after acceptor photobleaching (ErbB2/TR, left). As an experimental control copine III only was visualized with an Alexa488-labeled secondary antibody and FRET efficiency was measured after photobleaching (copine III only, right).

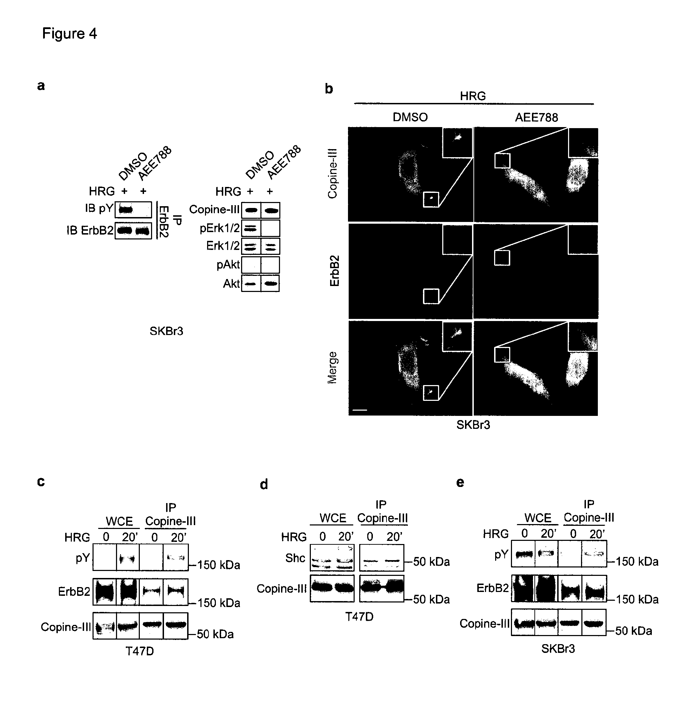

- **Figure 4:**: **The localization of copine III depends on activity of the ErbB2 receptor.** SKBr3 cells were starved overnight, treated with 0.5µM AEE788 or DMSO for controls for 1.5h and stimulated with HRG or PBS for controls for 20min. (**a**) Extracts of control and treated cells were taken for western blot analysis. ErbB2 was immunoprecipitated and its phosphorylation status was monitoring using a pTyr-specific antibody (a, left panel). Inhibition of downstream signaling pathways was monitored by western blotting of total lysates for Erk1/2, Akt and their phosphorylated forms. (**b**) The localization of copine III and ErbB2 in HRG treated SKBr3 control- and AEE788 treated cells was visualized by IF using their respective antibodies. Images of representative cells are shown (scale bar: 10µm). Inserts show 2x zooms of boxed regions focusing on the plasma membrane. (c, d and e) Co-immunoprecipitation of copine III, ErbB2 and Shc. Copine III was immunoprecipitated from lysates of control and HRG-stimulated T47D (c, d) or SKBr3 (e) cells using a specific monoclonal antibody. The co-IPs were probed for ErbB2 and pTyr (c, e) or for Shc (d). Whole cell extracts (WCE) are shown on the left of each panel.

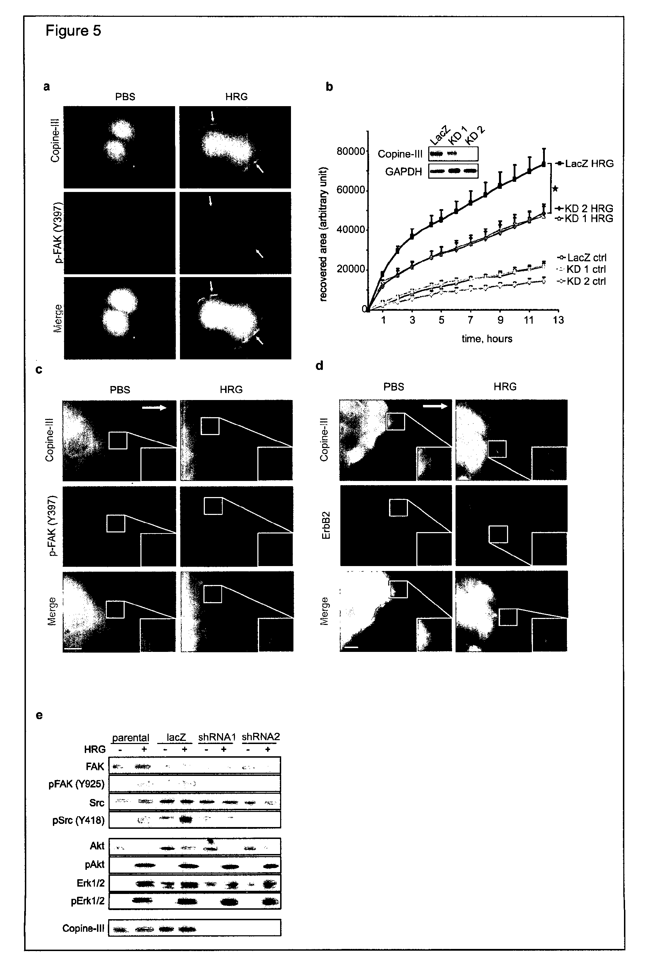

- **Figure 5:**: **Copine III has an important role in HRG-mediated T47D cell migration.** (**a**) Co-localization of copine III and phosphorylated focal adhesion kinase (pFAK). T47D cells were starved overnight and stimulated with 5nM HRG for 20min. Control cells were treated with PBS in parallel, Copine III and pTyr397FAK were visualized using their respective antibodies. Merged images in the bottom panel include Hoechst staining for the nucleus in blue (scale bar: 10µm). The arrows indicate areas of co-localization. (**b**) Wound healing assay in T47D copine III KD pools. Two pools of copine III KD T47D cells were generated with different shRNA targeting vectors (KD1 and 2). A control IacZ pool was also prepared. Expression levels of copine III in the KD1 and KD2 and the lacZ pools

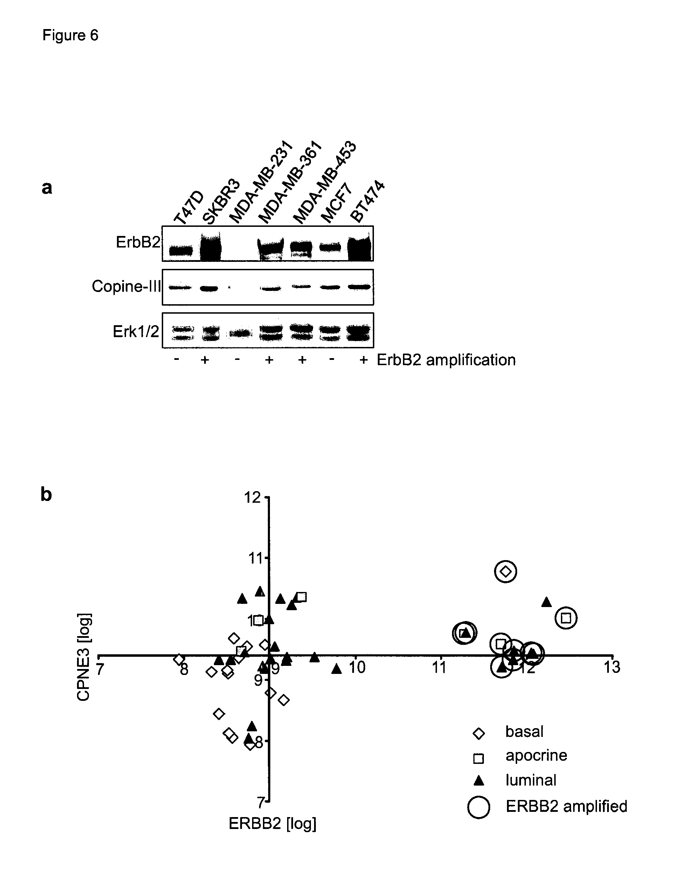

- **Figure 6:**: is shown; GAPDH is the loading control (insert). T47D cells were grown to confluency, starved in DMEM for 8h. Then cells were stimulated with 1 nM HRG and subsequently scratched subsequently. Cell migration into the wound was monitored for 12h in 4-6 regions of the scratch. The recovered area of the wound is shown by the mean ± SEM. * P < 0.005. (c and d) Localization of copine III and pTyr397FAK (c) and copine III and ErbB2 (d) in migrating T47D cells. Cells were grown to confluency, starved overnight and stimulated with 5nM HRG and scratched and allowed to migrate for 1.5h before they were fixed and stained. Copine III, pTyr397FAK and ErbB2 were visualized by IF using their respective antibodies. The direction of migration into the wound is indicated by the arrow. Merged images in the bottom panel include Hoechst staining for the nucleus in blue (scale bar: 10µm). Inserts show 2x zooms of boxed regions focusing on the leading edge of the migrating monolayer. (e) The two pools of copine III KD T47D cells, the control lacZ pool and the parental cells were treated or not with HRG for 20min. Cell lysates were prepared and western blots were probed for the level of FAK, pTyr925FAK, Src, pTyr481 Src, Akt, pAKT, Erk1/2, pErk1/2 and copine III **Copine III expression correlates with ErbB2 amplification in breast cancer.** (**a**) Protein expression levels of copine III and ErbB2 were analyzed in different breast cancer cell lines by western blotting. WCEs for each cell line were subjected to western blotting and probed for ErbB2, copine III and Erk1/2 as loading control. The ErbB2 amplification status is indicated by +/-. (**b**) Evaluation of *CPNE3* expression values in 49 primary breast tumor samples (Farmer *et al.,* 2005). Log expression of *CPNE3* is plotted on the x-axis and *ERBB2* log expression values on the y-axis. Axes are plotted through the median expression levels of *CPNE3* and *ERBB2,* respectively. The different subtypes of the analyzed breast tumors are denoted: Basal (◇), apocrine (▭) and luminal (▲). *ERBB2* amplified tumors are highlighted with circles.

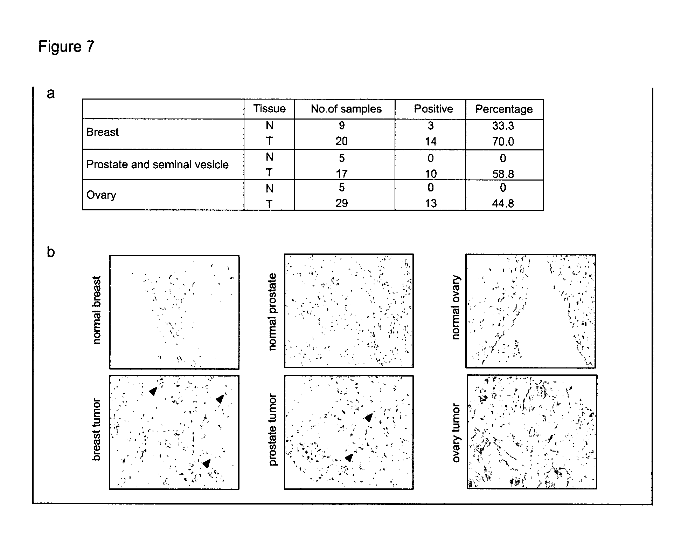

- **Figure 7:**: **Expression of Copine-III in breast, prostate and ovarian cancer samples.** (**a**) *CPNE3* expression analysis in different microarray datasets from the Oncomine database (www.oncomine.org) (Rhodes *et al.,* 2004). *CPNE3* RNA expression levels in samples from normal ovary and ovarian endometrioid adenocarcinoma samples from the Hendrix *et al.* dataset (Hendrix *et al.,* 2006). (**b**) Analysis of copine III expression in a human breast, prostate and ovarian tissue microarray (TMA). The table shows the number of samples analyzed for normal (N) and tumor tissue (T) for the respective cancer type. The number of copine III positive samples is annotated as well as their percentage from all analyzed samples using a cut-off of > 1.0 for breast and ≥1.0 for prostate and ovarian tissue samples. (c) Examples of IHC staining for copine III using the monoclonal antibody in breast, prostate and ovarian normal and cancer tissue samples. The magnification is 20x. The arrow heads indicate nuclear copine III staining.

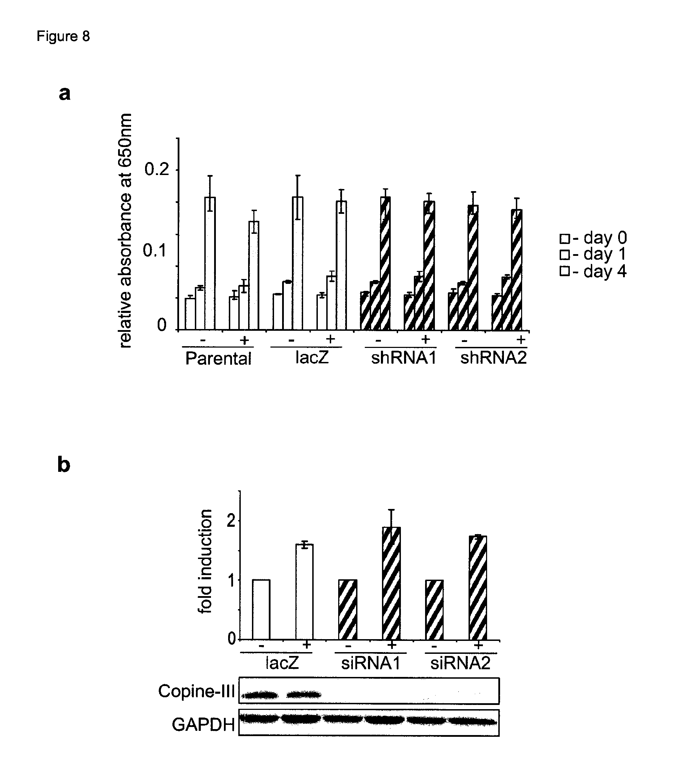

- **Figure 8:**: **Analysis of Copine-III KD cells.** (**a**) The relative proliferation of control and copine III KD T47D cell pools was monitored using the methylene blue assay as shown previously (Boulay *et* a/., 2008). Cell proliferation was measured in DMEM + 10% FBS for 4 days. The relative proliferation is shown by the absorbance at 650nm. (**b**) The potential for anchorage-independent growth was measured in T47D control and copine III KD (siRNA) cells. Cells were plated on polyhema-coated plates and allowed to grow without and in the presence of HRG (Boulay *et al.,* 2008). The fold induction of growth compared to the unstimulated cells is shown. The KD of copine III was verified by western blotting at the end of the experiment.

### Detailed Description of the Invention

The present inventors have now surprisingly found that the copine III interacts with activated ErbB2 at the plasma membrane of tumor cells, and is required for ErbB2-dependent cell migration.

The present invention hence provides a method for treating cancer in a subject by modulating copine III via the administration of a therapeutically effective amount of a modulator of copine III to said subject. In some embodiments, copine III is modulated by an inhibitor, such as an antibody. Alternatively, the inhibitor decreases or silences the expression of copine III, and is for instance a siRNA. In some embodiments, the subject is a mammal, for instance a human subject.

The methods of the invention are suitable for all cancers dependent on the activity of copine III. In one embodiment, the ErbB2-pathway is misregulated in the cells of the cancer. Example of such a misregulation are amplifications or overexpression of the *ErbB2* gene. In one embodiment, the cancer is a cancer of the breast, an ovarian cancer, a stomach cancer, or a uterine cancer, such as uterine serous endometrial carcinoma, which cancers are generally associated with a misregulation of the ErbB2-pathway. In some embodiments, the cancer is treated by inhibiting/reducing metastasis formation.

The present invention also encompasses a siRNA decreasing or silencing the expression of copine III or an antibody specifically binding to copine III for use as a medicament to treat cancer. Alternatively, the antibody can inhibit the binding of copine III to its ligands.

The present invention also provides methods of screening for agents able to modulate the expression of copine III expression and/or biological activity, which method comprises: (i) contacting a copine III polypeptide or a fragment thereof having the biological activity of copine III, a polynucleotide encoding such a polypeptide or polypeptide fragment, an expression vector comprising such a polynucleotide or a cell comprising such an expression vector, and a test substance under conditions that in the absence of the test substance would permit copine III expression and/or biological activity; and (ii) determining the amount of copine III expression and/or biological activity, to determine whether the test substance modulates copine III biological activity and/or expression, wherein a test substance which modulates copine III biological activity and/or expression is a potential therapeutical agent to treat cancer.

Moreover, the present invention also encompasses the modulators of the expression of copine III expression and/or of its biological activity identified using a method of screening of the invention.

In addition, the present invention also encompasses a method of diagnosing cancer comprising the step of assessing the level of expression of copine III in a sample from a subject.

These and other aspects of the present invention should be apparent to those skilled in the art, from the teachings herein.

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide. The term "isolated" does not refer to genomic or cDNA libraries, whole cell total or mRNA preparations, genomic DNA preparations (including those separated by electrophoresis and transferred onto blots), sheared whole cell genomic DNA preparations or other compositions where the art demonstrates no distinguishing features of the polynucleotide/sequences of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. However, a nucleic acid contained in a clone that is a member of a library (e.g., a genomic or cDNA library) that has not been isolated from other members of the library (e.g., in the form of a homogeneous solution containing the clone and other members of the library) or a chromosome removed from a cell or a cell lysate (e.g. , a "chromosome spread", as in a karyotype), or a preparation of randomly sheared genomic DNA or a preparation of genomic DNA cut with one or more restriction enzymes is not "isolated" for the purposes of this invention. As discussed further herein, isolated nucleic acid molecules according to the present invention may be produced naturally, recombinantly, or synthetically.

In the present invention, a "secreted" protein refers to a protein capable of being directed to the ER, secretory vesicles, or the extracellular space as a result of a signal sequence, as well as a protein released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

"Polynucleotides" can be composed of single-and double-stranded DNA, DNA that is a mixture of single-and double-stranded regions, single-and double-stranded RNA, and RNA that is mixture of single-and double-stranded regions, hybrid molecules comprising DNA and RNA that may be single-stranded or, more typically, double-stranded or a mixture of single-and double-stranded regions. In addition, polynucleotides can be composed of triple-stranded regions comprising RNA or DNA or both RNA and DNA. Polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The expression "polynucleotide encoding a polypeptide" encompasses a polynucleotide which includes only coding sequence for the polypeptide as well as a polynucleotide which includes additional coding and/or non-coding sequence.
"Stringent hybridization conditions" refers to an overnight incubation at 42 degree C in a solution comprising 50% formamide, 5x SSC (750 mM NaCl, 75 mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 50 degree C. Changes in the stringency of hybridization and signal detection are primarily accomplished through the manipulation of formamide concentration (lower percentages of formamide result in lowered stringency); salt conditions, or temperature. For example, moderately high stringency conditions include an overnight incubation at 37 degree C in a solution comprising 6X SSPE (20X SSPE = 3M NaCl; 0.2M NaH2PO4; 0.02M EDTA, pH 7.4), 0.5% SDS, 30% formamide, 100 µg/ml salmon sperm blocking DNA; followed by washes at 50 degree C with 1XSSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

The terms "fragment," "derivative" and "analog" when referring to polypeptides means polypeptides which either retain substantially the same biological function or activity as such polypeptides. An analog includes a proprotein which can be activated by cleavage of the proprotein portion to produce an active mature polypeptide.

The term "gene" means the segment of DNA involved in producing a polypeptide chain; it includes regions preceding and following the coding region "leader and trailer" as well as intervening sequences (introns) between individual coding segments (exons).

Polypeptides can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in the polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include, but are not limited to, acetylation, acylation, biotinylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, denivatization by known protecting/blocking groups, disulfide bond formation, demethylation, formation of covalent cross-links, formation of cysteine, formation of pyroglutamate, formylation, gamma-carboxylation, glycosylation, GPI anchor formation, hydroxylation, iodination, linkage to an antibody molecule or other cellular ligand, methylation, myristoylation, oxidation, pegylation, proteolytic processing (e.g., cleavage), phosphorylation, prenylation, racemization , selenoylation, sulfation, transfer-RNA mediated addition of amino acids to proteins such as arginylation, and ubiquitination.
(See, for instance, PROTEINS-STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. I-12 (1983); Seifter et al., Meth Enzymol 182:626-646 (1990); Rattan et al., Ann NY Acad Sci 663:48-62 (1992).)

A polypeptide fragment "having biological activity" refers to polypeptides exhibiting activity similar, but not necessarily identical to, an activity of the original polypeptide, including mature forms, as measured in a particular biological assay, with or without dose dependency. In the case where dose dependency does exist, it need not be identical to that of the polypeptide, but rather substantially similar to the dose-dependence in a given activity as compared to the original polypeptide (i.e., the candidate polypeptide will exhibit greater activity or not more than about 25-fold less and, in some embodiments, not more than about tenfold less activity, or not more than about three-fold less activity relative to the original polypeptide.)

Species homologs may be isolated and identified by making suitable probes or primers from the sequences provided herein and screening a suitable nucleic acid source for the desired homologue.

"Variant" refers to a polynucleotide or polypeptide differing from the original polynucleotide or polypeptide, but retaining essential properties thereof. Generally, variants are overall closely similar, and, in many regions, identical to the original polynucleotide or polypeptide.

As a practical matter, whether any particular nucleic acid molecule or polypeptide is at least 80%, 85%, 90%, 92%, 95%, 96%, 97%, 98%, 99%, or 100%identical to a nucleotide sequence of the present invention can be determined conventionally using known computer programs. A preferred method for determining the best overall match between a query sequence (a sequence of the present invention) and a subject sequence, also referred to as a global sequence aligmnent, can be determined using the FASTDB computer program based on the algorithm of Brutlag et al. (Comp. App. Blosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty--1, Joining Penalty--30, Randomization Group Length=0, Cutoff Score=l, Gap Penalty--5, Gap Size Penalty 0.05, Window Size=500 or the length of the subject nucleotide sequence, whichever is shorter. If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score. For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 impaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 92%, 95%, 96%, 97%, 98%, 99%, or 100% identical to, for instance, the amino acid sequences shown in a sequence or to the amino acid sequence encoded by deposited DNA clone can be determined conventionally using known computer programs. A preferred method for determining, the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty--I, Joining Penalty=20, Randomization Group Length=0, Cutoff Score=I, Window Size=sequence length, Gap Penalty--5, Gap Size Penalty--0.05, Window Size=500 or the length of the subject amino acid sequence, whichever is shorter. If the subject sequence is shorter than the query sequence due to N-or C-terminal deletions, not because of internal deletions, a manual correction must be made to the results. This is because the FASTDB program does not account for N-and C-terminal truncations of the subject sequence when calculating global percent identity. For subject sequences truncated at the N-and C-termini, relative to the query sequence, the percent identity is corrected by calculating the number of residues of the query sequence that are N-and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N-and C-termini of the subject sequence, which are not matched/aligned with the query sequence, are considered for the purposes of manually adjusting the percent identity score. That is, only query residue positions outside the farthest N-and C-terminal residues of the subject sequence. Only residue positions outside the N-and C-terminal ends of the subject sequence, as displayed in the FASTDB alignment, which are not matched/aligned with the query sequence are manually corrected for. No other manual corrections are to be made for the purposes of the present invention.

Naturally occurring protein variants are called "allelic variants," and refer to one of several alternate forms of a gene occupying a given locus on a chromosome of an organism. (Genes 11, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of polypeptides. For instance, one or more amino acids can be deleted from the N-terminus or C-terminus of a secreted protein without substantial loss of biological function. The authors of Ron et al., J. Biol. Chem. 268: 2984-2988 (1993), reported variant KGF proteins having hepanin binding activity even after deleting 3, 8, or 27 amino-terminal amino acid residues. Similarly, Interferon gamma exhibited up to ten times higher activity after deleting 8-10 amino acid residues from the carboxy terminus of this protein (Dobeli et al., J. Biotechnology 7:199-216 (1988)). Moreover, ample evidence demonstrates that variants often retain a biological activity similar to that of the naturally occurring protein. For example, Gayle and co-workers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[most of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type. Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N-or C-terminal residues of a protein retains such immunogenic activities can readily be determined by routine methods described herein and otherwise known in the art.

In one embodiment where one is assaying for the ability to bind or compete with full-length copine III polypeptide for binding to copine III antibody, various immunoassays known in the art can be used, including but not limited to, competitive and non-competitive assay systems using techniques such as radioimmunoassays, ELISA (enzyme linked immunosorbent assay), "sandwich" immunoassays, immunoradiometric assays, gel diffusion precipitation reactions, immunodiffasion assays, in situ immunoassays (using colloidal gold, enzyme or radioisotope labels, for example), western blots, precipitation reactions, agglutination assays (e.g., gel agglutination, assays, hemagglutination assays), complement fixation assays, immunofluorescence assays, protein A assays, and immunoelectrophoresis assays, etc. In one embodiment, antibody binding is detected by detecting a label on the primary antibody.

In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

Assays described herein and otherwise known in the art may routinely be applied to measure the ability of copine III polypeptides and fragments, variants derivatives and analogs thereof to elicit copine III-related biological activity (either *in vitro* or *in vivo*) and/or to assess whether copine III is present in a given sample, e.g. a sample isolated from a patient.

The term "epitopes," as used herein, refers to portions of a polypeptide having antigenic or immunogenic activity in an animal, in some embodiments, a mammal,for instance in a human. In an embodiment, the present invention encompasses a polypeptide comprising an epitope, as well as the polynucleotide encoding this polypeptide. An "immunogenic epitope," as used herein, is defined as a portion of a protein that elicits an antibody response in an animal, as determined by any method known in the art, for example, by the methods for generating antibodies described infra. (See, for example, Geysen et al., Proc. Natl. Acad. Sci. USA 81:3998-4002 (1983)). The term "antigenic epitope," as used herein, is defined as a portion of a protein to which an antibody can immuno specifically bind its antigen as determined by any method well known in the art, for example, by the immunoassays described herein. Immunospecific binding excludes non-specific binding but does not necessarily exclude cross-reactivity with other antigens. Antigenic epitopes need not necessarily be immunogenic.

Fragments which function as epitopes may be produced by any conventional means. (See, e.g., Houghten, Proc. Natl. Acad. Sci. USA 82:5131-5135 (1985), further described in U.S. Patent No. 4,631,211).

As one of skill in the art will appreciate, and as discussed above, polypeptides comprising an immunogenic or antigenic epitope can be fused to other polypeptide sequences. For example, polypeptides may be fused with the constant domain of immunoglobulins (lgA, IgE, IgG, IgM), or portions thereof (CHI, CH2, CH3, or any combination thereof and portions thereof), or albumin (including but not limited to recombinant albumin (see, e.g., U.S. Patent No. 5,876, 969, issued March 2, 1999, EP Patent 0 413 622, and U.S. Patent No. 5,766,883, issued June 16, 1998)), resulting in chimeric polypeptides. Such fusion proteins may facilitate purification and may increase half-life *in vivo.* This has been shown for chimeric proteins consisting of the first two domains of the human CD4-polypeptide and various domains of the constant regions of the heavy or light chains of mammalian immunoglobulins. See, e.g., EP 394,827; Traunecker et al., Nature, 331:84-86 (1988).

Enhanced delivery of an antigen across the epithelial barrier to the immune system has been demonstrated for antigens (e.g., insulin) conjugated to an FcRn binding partner such as IgG or Fc fragments (see, e. g., PCT Publications WO 96/22024 and WO 99/04813). IgG Fusion proteins that have a disulfide-linked dimeric structure due to the IgG portion disulfide bonds have also been found to be more efficient in binding and neutralizing other molecules than monomeric polypeptides or fragments thereof alone. See, e.g., Fountoulakis et al., J. Blochem., 270:3958-3964 (1995). Nucleic acids encoding the above epitopes can also be recombined with a gene of interest as an epitope tag (e.g., the hemagglutinin ("HA") tag or flag tag) to aid in detection and punification of the expressed polypeptide. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with the recombinant vaccinia virus are loaded onto Ni²⁺ nitriloacetic acidagarose column and histidine-tagged proteins can be selectively eluted with imidazole-containing buffers. Additional fusion proteins may be generated through the techniques of gene-shuffling, motif-shuffling, exon-shuffling, and/or codon-shuffling (collectively referred to as "DNA shuffling"). DNA shuffling may be employed to modulate the activities of polypeptides of the invention, such methods can be used to generate polypeptides with altered activity, as well as agonists and antagonists of the polypeptides. See, generally, U.S. Patent Nos. 5,605,793; 5,811,238; 5,830,721; 5,834, 252; and 5,837,458, and Patten et al., Curr. Opinion Biotechnol. 8:724-33 (1997); Harayama, Trends Biotechnol. 16(2):76-82 (1998); Hansson, et al., J. Mol. Biol. 287:265-76 (1999); and Lorenzo and Blasco, Biotechniques 24(2):308-13 (1998).

Antibodies of the invention include, but are not limited to, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules of the invention can be of any type (e.g., IgG, IgE, IgM, IgD, IgA and IgY), class (e.g., IgGI, IgG2, IgG3, IgG4, IgAI and IgA2) or subclass of immunoglobulin molecule. In addition, in the context of the present invention, the term "antibody" shall also encompass alternative molecules having the same function, e.g. aptamers and/or CDRs grafted onto alternative peptidic or non-peptidic frames.

In some embodiments the antibodies are human antigen-binding antibody fragments and include, but are not limited to, Fab, Fab' and F(ab')2, Fd, single-chain Fvs (scFv), single-chain antibodies, disulfide-linked Fvs (sdFv) and fragments comprising either a VL or VH domain. Antigen-binding antibody fragments, including single-chain antibodies, may comprise the variable region(s) alone or in combination with the entirety or a portion of the following: hinge region, CH1, CH2, and CH3 domains. Also included in the invention are antigen-binding fragments also comprising any combination of variable region(s) with a hinge region, CH1, CH2, and CH3 domains. The antibodies of the invention may be from any animal origin including birds and mammals. In some embodiments, the antibodies are human, murine (e.g., mouse and rat), donkey, ship rabbit, goat, guinea pig, camel, shark, horse, or chicken. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example in, U.S. Patent No. 5,939,598 by Kucherlapati et al. The antibodies of the present invention may be monospecific, bispecific, trispecific or of greater multi specificity. Multispecific antibodies may be specific for different epitopes of a polypeptide or may be specific for both a polypeptide as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Patent Nos. 4, 474,893; 4,714,681; 4,925,648; 5,573,920; 5,601,819; Kostelny et al., J. Immunol. 148:1547-1553 (1992).

Antibodies of the present invention may be described or specified in terms of the epitope(s) or portion(s) of a polypeptide which they recognize or specifically bind. The epitope(s) or polypeptide portion(s) may be specified as described herein, e.g., by N-terminal and C-terminal positions, by size in contiguous amino acid residues.

Antibodies may also be described or specified in terms of their cross-reactivity. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide are also included in the present invention. In specific embodiments, antibodies of the present invention cross-react with murine, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%. less than 55%, and less than 50% identity (as calculated using methods known in the art and described herein) to a polypeptide are also included in the present invention.
Antibodies may also be described or specified in terms of their binding affinity to a polypeptide Antibodies may act as agonists or antagonists of the recognized polypeptides. The invention also features receptor-specific antibodies which do not prevent ligand binding but prevent receptor activation. Receptor activation (i.e., signalling) may be determined by techniques described herein or otherwise known in the art. For example, receptor activation can be determined by detecting the phosphorylation (e.g., tyrosine or serine/threonine) of the receptor or of one of its down-stream substrates by immunoprecipitation followed by western blot analysis (for example, as described supra). In specific embodiments, antibodies are provided that inhibit ligand activity or receptor activity by at least 95%, at least 90%, at least 85%, at least 80%, at least 75%, at least 70%, at least 60%, or at least 50% of the activity in absence of the antibody.

The invention also features receptor-specific antibodies which both prevent ligand binding and receptor activation as well as antibodies that recognize the receptor-ligand complex. Likewise, encompassed by the invention are antibodies which bind the ligand, thereby preventing receptor activation, but do not prevent the ligand from binding the receptor. The antibodies may be specified as agonists, antagonists or inverse agonists for biological activities comprising the specific biological activities of the peptides disclosed herein. The above antibody agonists can be made using methods known in the art. See, e.g., PCT publication WO 96/40281; U.S. Patent No. 5,811, 097; Deng et al., Blood 92(6):1981-1988 (1998); Chen et al., Cancer Res. 58(16):3668-3678 (1998); Harrop et al., J. Immunol. 161(4):1786-1794 (1998); Zhu et al., Cancer Res. 58(15):3209-3214 (1998); Yoon et al., J. Immunol. 160(7):3170-3179 (1998); Prat et al., J. Cell. Sci. III(Pt2) :237-247 (1998); Pitard et al., J. Immunol. Methods 205(2):177-190 (1997); Liautard et al., Cytokine 9(4):233-241 (1997); Carlson et al., J. Biol. Chem. 272(17):11295-11301 (1997); Taryman et al., Neuron 14(4):755-762 (1995); Muller et al., Structure 6(9):1153-1167 (1998); Bartunek et al., Cytokine 8(I):14-20 (1996).

As discussed in more detail below, the antibodies may be used either alone or in combination with other compositions. The antibodies may further be recombinantly fused to a heterologous polypeptide at the N-or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs, radionuclides, or toxins. See, e.g., PCT publications WO 92/08495; WO 91/14438; WO 89/12624; U.S. Patent No. 5,314,995; and EP 396, 387.

The antibodies as defined for the present invention include derivatives that are modified, i. e, by the covalent attachment of any type of molecule to the antibody such that covalent attachment does not prevent the antibody from generating an anti-idiotypic response. For example, but not by way of limitation, the antibody derivatives include antibodies that have been modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

The antibodies of the present invention may be generated by any suitable method known in the art. Polyclonal antibodies to an antigen-of-interest can be produced by various procedures well known in the art. For example, a polypeptide of the invention can be administered to various host animals including, but not limited to, rabbits, mice, rats, etc. to induce the production of sera containing polyclonal antibodies specific for the antigen.

Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvurn. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art.

Antibody fragments which recognize specific epitopes may be generated by known techniques. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain.

For example, the antibodies can also be generated using various phage display methods known in the art. In phage display methods, functional antibody domains are displayed on the surface of phage particles which carry the polynucleotide sequences encoding them. In a particular embodiment, such phage can be utilized to display antigen binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Examples of phage display methods that can be used to make the antibodies of the present invention include those disclosed in Brinkman et al., J. lmmunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publications WO 90/02809; WO 91/10737; WO 92/01047; WO 92/18619; WO 93/11236; WO 95/15982; WO 95/20401; and U.S. Patent Nos. 5, 698,426; 5,223,409; 5,403,484; 5,580,717; 5,427,908; 5,750,753; 5,821, 047; 5,571, 698; 5,427,908; 5,516,637; 5,780,225; 5,658,727; 5,733,743 and 5,969,108. As described in these references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in PCT publication WO 92/22324; Mullinax. et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988). For some uses, including in vivo use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. See e.g., Morrison, Science 229:1202 (1985); Oi et al., BioTechniques 4:214 (1986); Gillies et al., (1989) J. Immunol. Methods 125:191-202; U.S. Patent Nos. 5,807,715; 4,816,567; and 4,816397. Humanized antibodies are antibody molecules from non-human species antibody that binds the desired antigen having one or more complementarity determining regions (CDRs) from the non-human species and a framework regions from a human immunoglobulin molecule. Often, framework residues in the human framework regions will be substituted with the corresponding residue from the CDR donor antibody to alter, and/or improve, antigen binding. These framework substitutions are identified by methods well known in the art, e.g., by modelling of the interactions of the CDR and framework residues to identify framework residues important for antigen binding and sequence comparison to identify unusual framework residues at particular positions. (See, e.g., Queen et al., U.S. Patent No. 5,585,089; Riechmann et al., Nature 332:323 (1988).) Antibodies can be humanized using a variety of techniques known in the art including, for example, CDR-grafting (EP 239,400; PCT publication WO 91/09967; U.S. Patent Nos. 5,225,539; 5,530,101; and 5,585,089), veneering or resurfacing (EP 592, 106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Patent No. 5,565,332).

Completely human antibodies are particularly desirable for therapeutic treatment of human patients. Human antibodies can be made by a variety of methods known in the art including phage display methods described above using antibody libraries derived from human immunoglobulin sequences. See also, U.S. Patent Nos. 4,444,887 and 4,716, 111; and PCT publications WO 98/46645, WO 98/50433, WO 98/24893, WO 98/16654, WO 96/34096, WO 96/33735, and WO 91/10741.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, e.g., all or a portion of a polypeptide of the invention. Monoclonal antibodies directed against the antigen can be obtained from the immunized, transgenic mice using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar, Int. Rev. Immurnol. 13:65-93 (1995). For a detailed discussion of this technology for producing human antibodies and human monoclonal antibodies and protocols for producing such antibodies, see, e. g., PCT publications WO 98/24893; WO 92/01047; WO 96/34096; WO 96/33735; European Patent No. 0 598 877; U.S. Patent Nos. 5,413,923; 5,625,126; 5,633,425; 5,569, 825; 5, 661,016; 5,545,806; 5,814,318; 5,885,793; 5,916,771; and 5,939,598. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology 12:899-903 (1988)).

Furthermore, antibodies can be utilized to generate anti-idiotype antibodies that "mimic" polypeptides using techniques well known to those skilled in the art. (See, e.g., Greenspan & Bona, FASEB J. 7(5):437-444; (1989) and Nissinoff, J. Immunol. 147(8):2429-2438 (1991)). For example, antibodies which bind to and competitively inhibit polypeptide multimerization. and/or binding of a polypeptide to a ligand can be used to generate anti-idiotypes that "mimic" the polypeptide multimerization. and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide and/or to bind its ligands/receptors, and thereby block its biological activity.

Polynucleotides encoding antibodies, comprising a nucleotide sequence encoding an antibody are also encompassed. These polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

The amino acid sequence of the heavy and/or light chain variable domains may be inspected to identify the sequences of the complementarity determining regions (CDRs) by methods that are well know in the art, e.g., by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody, as described supra. The framework regions may be naturally occurring or consensus framework regions, and in some embodiments, human framework regions (see, e.g., Chothia et al., J. Mol. Biol. 278: 457-479 (1998) for a listing of human framework regions). In some embodiments, the polynucleotide generated by the combination of the framework regions and CDRs encodes an antibody that specifically binds a polypeptide. In some embodiments, as discussed supra, one or more amino acid substitutions may be made within the framework regions, and, in some embodiments, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polymicleotide are encompassed by the present description and within the skill of the art.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human immunoglobulin constant region, e.g., humanized antibodies.

Alternatively, techniques described for the production of single chain antibodies (U.S. Patent No. 4,946,778; Bird, Science 242:423-42 (1988); Huston et al., Proc. Natl. Acad. Sci. USA 85:5879-5883 (1988); and Ward et al., Nature 334:544-54 (1989)) can be adapted to produce single chain antibodies. Single chain antibodies are formed by linking the heavy and light chain fragments of the Fv region via an amino acid bridge, resulting in a single chain polypeptide. Techniques for the assembly of functional Fv fragments in E. coli may also be used (Skerra et al., Science 242:1038-1041 (1988)).

The present invention encompasses antibodies recombinantly fused or chemically conjugated (including both covalently and non-covalently conjugations) to a polypeptide (or portion thereof, in some embodiments, at least 10, 20, 30, 40, 50, 60, 70, 80, 90 or 100 amino acids of the polypeptide) to generate fusion proteins. The fusion does not necessarily need to be direct, but may occur through linker sequences. The antibodies may be specific for antigens other than polypeptides (or portion thereof, in some embodiments, at least 10, 20, 30, 40, 50, 60, 70, 80, 90 or 100 amino acids of the polypeptide).

Further, an antibody or fragment thereof may be conjugated to a therapeutic moiety, for instance to increase their therapeutical activity. The conjugates can be used for modifying a given biological response, the therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, a-interferon, B-interferon, nerve growth factor, platelet derived growth factor, tissue plasminogen activator, an apoptotic agent, e.g., TNF-alpha, TNF-beta, AIM I (See, International Publication No. WO 97/33899), AIM 11 (See, International Publication No. WO 97/34911), Fas Ligand (Takahashi et aL, Int. Immunol., 6:1567-1574 (1994)), VEGI (See, International Publication No. WO 99/23105) , a thrombotic agent or an anti-angiogenic agent, e.g., angiostatin or endostatin; or, biological response modifiers such as, for example, lymphokines, interleukin-1 ("IL-I"), interleukin-2 ("IL-2"), interleukin-6 ("IL-6"), granulocyte macrophage colony stimulating factor ("GM-CSF"), granulocyte colony stimulating factor ("G-CSF"), or other growth factors. Techniques for conjugating such therapeutic moiety to antibodies are well known, see, e.g., Amon et al., "Monoclonal Antibodies For lmmunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev. 62:119-58 (1982).

Alternatively, an antibody can be conjugated to a second antibody to form an antibody heteroconjugate as described by Segal in U.S. Patent No. 4,676, 980.

The present invention is also directed to antibody-based therapies which involve administering antibodies of the invention to an animal, in some embodiments, a mammal, for example a human, patient to treat cancer. Therapeutic compounds include, but are not limited to, antibodies (including fragments, analogs and derivatives thereof as described herein) and nucleic acids encoding antibodies of the invention (including fragments, analogs and derivatives thereof and anti-idiotypic antibodies as described herein). Antibodies of the invention may be provided in pharmaceutically acceptable compositions as known in the art or as described herein.

The invention also provides methods for treating cancer in a subject by inhibiting copine III by administration to the subject of an effective amount of an inhibitory compound or pharmaceutical composition comprising such inhibitory compound. In some embodiments, said inhibitory compound is an antibody or an siRNA. In an embodiment, the compound is substantially purified (e.g., substantially free from substances that limit its effect or produce undesired side-effects). The subject is in some embodiments, an animal, including but not limited to animals such as cows, pigs, horses, chickens, cats, dogs, etc., and is in some embodiments, a mammal, for example human.

Formulations and methods of administration that can be employed when the compound comprises a nucleic acid or an immunoglobulin are described above; additional appropriate formulations and routes of administration can be selected from among those described herein below.

Various delivery systems are known and can be used to administer a compound, e.g., encapsulation in liposomes, microparticles, microcapsules, recombinant cells capable of expressing the compound, receptor-mediated endocytosis (see, e. g., Wu and Wu, J. Biol. Chem. 262:4429-4432 (1987)), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers.

In another embodiment, the compound or composition can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.) In yet another embodiment, the compound or composition can be delivered in a controlled release system. In one embodiment, a pump may be used (see Langer, supra; Sefton, CRC Crit. Ref, Biomed. Eng. 14:201 (1987); Buchwald et al., Surgery 88:507 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, i.e., the brain, thus requiring only a fraction of the systemic dose (see, e.g. , Goodson, in Medical Applications of Controlled Release, supra, vol. 2, pp. 115-13 8 (1984)).

Other controlled release systems are discussed in the review by Langer (Science 249:1527-1533 (1990)).

The present invention also provides pharmaceutical compositions for use in the treatment of cancer by inhibiting a copine III. Such compositions comprise a therapeutically effective amount of an inhibitory compound, and a pharmaceutically acceptable carrier. In a specific embodiment, the term "pharmaceutically acceptable" means approved by a regulatory agency of the Federal or a state government or listed in the U. S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, tale, sodium chloride, driied skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, in some embodiments, in purified form, together with a suitable amount of carrier so as to provide the form for proper administration to the patient. The formulation should suit the mode of administration.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection.

Generally, the ingredients are supplied either separately or mixed together in unit dosage form, for example, as a dry lyophilized powder or water free concentrate in a hermetically scaled container such as an ampoule or sachette indicating the quantity of active agent.

Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms.

Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc. The amount of the compound which will be effective in the treatment, inhibition and prevention of a disease or disorder associated with aberrant expression and/or activity of a polypeptide of the invention can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances.

Effective doses may be extrapolated from dose-response curves derived from *in vitro* or animal model test systems.

For antibodies, the dosage administered to a patient is typically 0.1 mg/kg to 100 mg/kg of the patient's body weight. In some embodiments, the dosage administered to a patient is between 0.1 mg/kg and 20 mg/kg of the patient's body weight, for example1 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention may be reduced by enhancing uptake and tissue penetration (e.g., into the brain) of the antibodies by modifications such as, for example, lipidation.

Also encompassed is a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The antibodies as encompassed herein may also be chemically modified derivatives which may provide additional advantages such as increased solubility, stability and circulating time of the polypeptide, or decreased immunogenicity (see U. S. Patent No. 4,179,337). The chemical moieties for derivatisation may be selected from water soluble polymers such as polyethylene glycol, ethylene glycol/propylene glycol copolymers, carboxymethy1cellulose, dextran, polyvinyl alcohol and the like. The antibodies may be modified at random positions within the molecule, or at predetermined positions within the molecule and may include one, two, three or more attached chemical moieties. The polymer may be of any molecular weight, and may be branched or unbranched. For polyethylene glycol, the preferred molecular weight is between about 1 kDa and about 100000 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,600, 17,500, 18,000, 18,500, 19,000, 19,500, 20,000, 25,000, 30,000, 35,000, 40,000, 50,000, 55,000, 60,000, 65,000, 70,000, 75,000, 80,000, 85,000, 90,000, 95,000, or 100,000 kDa. As noted above, the polyethylene glycol may have a branched structure. Branched polyethylene glycols are described, for example, in U. S. Patent No. 5,643, 575; Morpurgo et al., Appl. Biochem. Biotechnol. 56:59-72 (1996); Vorobjev et al., Nucleosides Nucleotides 18:2745-2750 (1999); and Caliceti et al., Bioconjug. Chem. 10:638-646 (1999). The polyethylene glycol molecules (or other chemical moieties) should be attached to the protein with consideration of effects on functional or antigenic domains of the protein. There are a number of attachment methods available to those skilled in the art, e.g., EP 0 401 384 (coupling PEG to G-CSF), see also Malik et al., Exp. Hematol. 20:1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group. As suggested above, polyethylene glycol may be attached to proteins via linkage to any of a number of amino acid residues. For example, polyethylene glycol can be linked to proteins via covalent bonds to lysine, histidine, aspartic acid, glutamic acid, or cysteine residues. One or more reaction chemistries may be employed to attach polyethylene glycol to specific amino acid residues (e.g., lysine, histidine, aspartic acid, glutamic acid, or cysteine) of the protein or to more than one type of amino acid residue (e.g., lysine, histidine, aspartic acid, glutamic acid, cysteine and combinations thereof) of the protein. As indicated above, pegylation of the proteins of the invention may be accomplished by any number of means. For example, polyethylene glycol may be attached to the protein either directly or by an intervening linker. Linkerless systems for attaching polyethylene glycol to proteins are described in Delgado et al., Crit. Rev. Thera. Drug Carrier Sys. 9:249-304 (1992); Francis et al., Intern. J. of Hematol. 68:1-18 (1998); U.S. Patent No. 4,002,53 1; U.S. Patent No. 5,349,052; WO 95/06058; and WO 98/32466.

By "biological sample" is intended any biological sample obtained from an individual, body fluid, cell line, tissue culture, or other source which contains the polypeptide of the present invention or mRNA. As indicated, biological samples include body fluids (such as semen, lymph, sera, plasma, urine, synovial fluid and spinal fluid) which contain the polypeptide of the present invention, and other tissue sources found to express the polypeptide of the present invention. Methods for obtaining tissue biopsies and body fluids from mammals are well known in the art. Where the biological sample is to include mRNA, a tissue biopsy is the preferred source.

"RNAi" is the process of sequence specific post-transcriptional gene silencing in animals and plants. It uses small interfering RNA molecules (siRNA) that are double-stranded and homologous in sequence to the silenced (target) gene. Hence, sequence specific binding of the siRNA molecule with mRNAs produced by transcription of the target gene allows very specific targeted knockdown' of gene expression.

"siRNA" or "small-interfering ribonucleic acid" according to the invention has the meanings known in the art, including the following aspects. The siRNA consists of two strands of ribonucleotides which hybridize along a complementary region under physiological conditions. The strands are normally separate. Because of the two strands have separate roles in a cell, one strand is called the "antisense" strand, also known as the "guide" sequence, and is used in the functioning RISC complex to guide it to the correct mRNA for cleavage. This use of "anti-sense", because it relates to an RNA compound, is different from the antisense target DNA compounds referred to elsewhere in this specification. The other strand is known as the "anti-guide" sequence and because it contains the same sequence of nucleotides as the target sequence, it is also known as the sense strand. The strands may be joined by a molecular linker in certain embodiments. The individual ribonucleotides may be unmodified naturally occurring ribonucleotides, unmodified naturally occurring deoxyribonucleotides or they may be chemically modified or synthetic as described elsewhere herein.

In some embodiments, the siRNA molecule is substantially identical with at least a region of the coding sequence of the target gene to enable down-regulation of the gene. In some embodiments, the degree of identity between the sequence of the siRNA molecule and the targeted region of the gene is at least 60% sequence identity, in some embodiments at least 75% sequence identity, for instance at least 85% identity, 90% identity, at least 95% identity, at least 97%, or at least 99% identity.

Calculation of percentage identities between different amino acid/polypeptide/nucleic acid sequences may be carried out as follows. A multiple alignment is first generated by the ClustalX program (pairwise parameters: gap opening 10.0, gap extension 0.1, protein matrix Gonnet 250, DNA matrix IUB; multiple parameters: gap opening 10.0, gap extension 0.2, delay divergent sequences 30%, DNA transition weight 0.5, negative matrix off, protein matrix gonnet series, DNA weight IUB; Protein gap parameters, residue-specific penalties on, hydrophilic penalties on, hydrophilic residues GPSNDQERK, gap separation distance 4, end gap separation off). The percentage identity is then calculated from the multiple alignment as (N/T)* 100, where N is the number of positions at which the two sequences share an identical residue, and T is the total number of positions compared. Alternatively, percentage identity can be calculated as (N/S)* 100 where S is the length of the shorter sequence being compared. The amino acid/polypeptide/nucleic acid sequences may be synthesised de novo, or may be native amino acid/polypeptide/nucleic acid sequence, or a derivative thereof. A substantially similar nucleotide sequence will be encoded by a sequence which hybridizes to any of the nucleic acid sequences referred to herein or their complements under stringent conditions. By stringent conditions, we mean the nucleotide hybridises to filter-bound DNA or RNA in 6x sodium chloride/sodium citrate (SSC) at approximately 45°C followed by at least one wash in 0.2x SSC/0.1% SDS at approximately 5-65°C. Alternatively, a substantially similar polypeptide may differ by at least 1, but less than 5, 10, 20, 50 or 100 amino acids from the peptide sequences according to the present invention Due to the degeneracy of the genetic code, it is clear that any nucleic acid sequence could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequences which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine; large non-polar, hydrophobic amino acids include phenylalanine, tryptophan and tyrosine; the polar neutral amino acids include serine, threonine, cysteine, asparagine and glutamine; the positively charged (basic) amino acids include lysine, arginine and histidine; and the negatively charged (acidic) amino acids include aspartic acid and glutamic acid.

The accurate alignment of protein or DNA sequences is a complex process, which has been investigated in detail by a number of researchers. Of particular importance is the trade-off between optimal matching of sequences and the introduction of gaps to obtain such a match. In the case of proteins, the means by which matches are scored is also of significance. The family of PAM matrices (e.g., Dayhoff, M. et al., 1978, Atlas of protein sequence and structure, Natl. Biomed. Res. Found.) and BLOSUM matrices quantify the nature and likelihood of conservative substitutions and are used in multiple alignment algorithms, although other, equally applicable matrices will be known to those skilled in the art. The popular multiple alignment program ClustalW, and its windows version ClustalX (Thompson et al., 1994, Nucleic Acids Research, 22, 4673-4680; Thompson et al., 1997, Nucleic Acids Research, 24, 4876-4882) are efficient ways to generate multiple alignments of proteins and DNA.

Frequently, automatically generated alignments require manual alignment, exploiting the trained user's knowledge of the protein family being studied, e.g., biological knowledge of key conserved sites. One such alignment editor programs is Align (http://www.gwdg.de/dhepper/download/; Hepperle, D., 2001: Multicolor Sequence Alignment Editor. Institute of Freshwater Ecology and Inland Fisheries, 16775 Stechlin, Germany), although others, such as JalView or Cinema are also suitable.

Calculation of percentage identities between proteins occurs during the generation of multiple alignments by Clustal. However, these values need to be recalculated if the alignment has been manually improved, or for the deliberate comparison of two sequences. Programs that calculate this value for pairs of protein sequences within an alignment include PROTDIST within the PHYLIP phylogeny package (Felsenstein; http://evolution.gs.washington.edu/ phylip.html) using the "Similarity Table" option as the model for amino acid substitution (P). For DNA/RNA, an identical option exists within the DNADIST program of PHYL1 P.

The dsRNA molecules in accordance with the present invention comprise a double-stranded region which is substantially identical to a region of the mRNA of the target gene. A region with 100% identity to the corresponding sequence of the target gene is suitable. This state is referred to as "fully complementary". However, the region may also contain one, two or three mismatches as compared to the corresponding region of the target gene, depending on the length of the region of the mRNA that is targeted, and as such may be not fully complementary. In an embodiment, the RNA molecules of the present invention specifically target one given gene. In order to only target the desired mRNA, the siRNA reagent may have 100% homology to the target mRNA and at least 2 mismatched nucleotides to all other genes present in the cell or organism. Methods to analyze and identify siRNAs with sufficient sequence identity in order to effectively inhibit expression of a specific target sequence are known in the art. Sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group).

The length of the region of the siRNA complementary to the target, in accordance with the present invention, may be from 10 to 100 nucleotides, 12 to 25 nucleotides, 14 to 22 nucleotides or 15, 16, 17 or 18 nucleotides. Where there are mismatches to the corresponding target region, the length of the complementary region is generally required to be somewhat longer. In an embodiment, the inhibitor is a siRNA molecule and comprises between approximately 5bp and 50 bp, in some embodiments, between 10 bp and 35 bp, or between 15 bp and 30 bp, for instance between 18 bp and 25bp. In some embodiments, the siRNA molecule comprises more than 20 and less than 23 bp.

Because the siRNA may carry overhanging ends (which may or may not be complementary to the target), or additional nucleotides complementary to itself but not the target gene, the total length of each separate strand of siRNA may be 10 to 100 nucleotides, 15 to 49 nucleotides, 17 to 30 nucleotides or 19 to 25 nucleotides.

The phrase "each strand is 49 nucleotides or less" means the total number of consecutive nucleotides in the strand, including all modified or unmodified nucleotides, but not including any chemical moieties which may be added to the 3' or 5' end of the strand. Short chemical moieties inserted into the strand are not counted, but a chemical linker designed to join two separate strands is not considered to create consecutive nucleotides.

The phrase "a 1 to 6 nucleotide overhang on at least one of the 5' end or 3' end" refers to the architecture of the complementary siRNA that forms from two separate strands under physiological conditions. If the terminal nucleotides are part of the double-stranded region of the siRNA, the siRNA is considered blunt ended. If one or more nucleotides are unpaired on an end, an overhang is created. The overhang length is measured by the number of overhanging nucleotides. The overhanging nucleotides can be either on the 5' end or 3' end of either strand.

The siRNA according to the present invention display a high in vivo stability and may be particularly suitable for oral delivery by including at least one modified nucleotide in at least one of the strands. Thus the siRNA according to the present invention contains at least one modified or non-natural ribonucleotide. A lengthy description of many known chemical modifications are set out in published PCT patent application WO 200370918. Suitable modifications for delivery include chemical modifications can be selected from among:
a) a 3' cap;
b) a 5' cap,
c) a modified internucleoside linkage; or
d) a modified sugar or base moiety.

Suitable modifications include, but are not limited to modifications to the sugar moiety (i.e. the 2' position of the sugar moiety, such as for instance 2'-O-(2-methoxyethyl) or 2'-MOE) (Martin et al., Helv. Chim. Acta, 1995, 78, 486-504) i.e., an alkoxyalkoxy group) or the base moiety (i.e. a non-natural or modified base which maintains ability to pair with another specific base in an alternate nucleotide chain). Other modifications include so-called 'backbone' modifications including, but not limited to, replacing the phosphoester group (connecting adjacent ribonucleotides) with for instance phosphorothioates, chiral phosphorothioates or phosphorodithioates.

End modifications sometimes referred to herein as 3' caps or 5' caps may be of significance. Caps may consist of simply adding additional nucleotides, such as "T-T" which has been found to confer stability on a siRNA. Caps may consist of more complex chemistries which are known to those skilled in the art.

Design of a suitable siRNA molecule is a complicated process, and involves very carefully analysing the sequence of the target mRNA molecule. On exemplary method for the design of siRNA is illustrated in W02005/059132. Then, using considerable inventive endeavour, the inventors have to choose a defined sequence of siRNA which has a certain composition of nucleotide bases, which would have the required affinity and also stability to cause the RNA interference.

The siRNA molecule may be either synthesised de novo, or produced by a micro-organism. For example, the siRNA molecule may be produced by bacteria, for example, E. coli. Methods for the synthesis of siRNA, including siRNA containing at least one modified or non-natural ribonucleotides are well known and readily available to those of skill in the art. For example, a variety of synthetic chemistries are set out in published PCT patent applications WO2005021749 and WO200370918. The reaction may be carried out in solution or, in some embodiments, on solid phase or by using polymer supported reagents, followed by combining the synthesized RNA strands under conditions, wherein a siRNA molecule is formed, which is capable of mediating RNAi.

It should be appreciated that siNAs (small interfering nucleic acids) may comprise uracil (siRNA) or thyrimidine (siDNA). Accordingly the nucleotides U and T, as referred to above, may be interchanged. However it is preferred that siRNA is used.

Gene-silencing molecules, i.e. inhibitors, used according to the invention are in some embodiments, nucleic acids (e.g. siRNA or antisense or ribozymes). Such molecules may (but not necessarily) be ones, which become incorporated in the DNA of cells of the subject being treated. Undifferentiated cells may be stably transformed with the gene-silencing molecule leading to the production of genetically modified daughter cells (in which case regulation of expression in the subject may be required, e.g. with specific transcription factors, or gene activators).

The gene-silencing molecule may be either synthesised *de novo,* and introduced in sufficient amounts to induce gene-silencing (e.g. by RNA interference) in the target cell. Alternatively, the molecule may be produced by a micro-organism, for example, E. coli, and then introduced in sufficient amounts to induce gene silencing in the target cell.

The molecule may be produced by a vector harbouring a nucleic acid that encodes the gene-silencing sequence. The vector may comprise elements capable of controlling and/or enhancing expression of the nucleic acid. The vector may be a recombinant vector. The vector may for example comprise plasmid, cosmid, phage, or virus DNA. In addition to, or instead of using the vector to synthesise the gene-silencing molecule, the vector may be used as a delivery system for transforming a target cell with the gene silencing sequence.

The recombinant vector may also include other functional elements. For instance, recombinant vectors can be designed such that the vector will autonomously replicate in the target cell. In this case, elements that induce nucleic acid replication may be required in the recombinant vector. Alternatively, the recombinant vector may be designed such that the vector and recombinant nucleic acid molecule integrates into the genome of a target cell. In this case nucleic acid sequences, which favour targeted integration (e.g. by homologous recombination) are desirable. Recombinant vectors may also have DNA coding for genes that may be used as selectable markers in the cloning process.

The recombinant vector may also comprise a promoter or regulator or enhancer to control expression of the nucleic acid as required. Tissue specific promoter/enhancer elements may be used to regulate expression of the nucleic acid in specific cell types, for example, endothelial cells. The promoter may be constitutive or inducible.

Alternatively, the gene silencing molecule may be administered to a target cell or tissue in a subject with or without it being incorporated in a vector. For instance, the molecule may be incorporated within a liposome or virus particle (e.g. a retrovirus, herpes virus, pox virus, vaccina virus, adenovirus, lentivirus and the like).

Alternatively a "naked" siRNA or antisense molecule may be inserted into a subject's cells by a suitable means e.g. direct endocytotic uptake.

The gene silencing molecule may also be transferred to the cells of a subject to be treated by either transfection, infection, microinjection, cell fusion, protoplast fusion or ballistic bombardment. For example, transfer may be by: ballistic transfection with coated gold particles; liposomes containing a siNA molecule; viral vectors comprising a gene silencing sequence or means of providing direct nucleic acid uptake (e.g. endocytosis) by application of the gene silencing molecule directly.

In an embodiment of the present invention siNA molecules may be delivered to a target cell (whether in a vector or "naked") and may then rely upon the host cell to be replicated and thereby reach therapeutically effective levels. When this is the case the siNA is in some embodiments, incorporated in an expression cassette that will enable the siNA to be transcribed in the cell and then interfere with translation (by inducing destruction of the endogenous mRNA coding the targeted gene product).

Inhibitors according to any embodiment of the present invention may be used in a monotherapy (e.g. use of siRNAs alone). However it will be appreciated that the inhibitors may be used as an adjunct, or in combination with other therapies.

The inhibitors of copine III may be contained within compositions having a number of different forms depending, in particular on the manner in which the composition is to be used. Thus, for example, the composition may be in the form of a capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given, and in some embodiments, enables delivery of the inhibitor to the target site.

The inhibitors of copine III may be used in a number of ways.

For instance, systemic administration may be required in which case the compound may be contained within a composition that may, for example, be administered by injection into the blood stream. Injections may be intravenous (bolus or infusion), subcutaneous, intramuscular or a direct injection into the target tissue (e.g. an intraventricular injection-when used in the brain). The inhibitors may also be administered by inhalation (e.g. intranasally) or even orally (if appropriate).

The inhibitors of the invention may also be incorporated within a slow or delayed release device. Such devices may, for example, be inserted at the site of a tumour, and the molecule may be released over weeks or months. Such devices may be particularly advantageous when long term treatment with an inhibitor of copine III is required and which would normally require frequent administration (e.g. at least daily injection).

It will be appreciated that the amount of an inhibitor that is required is determined by its biological activity and bioavailability which in turn depends on the mode of administration, the physicochemical properties of the molecule employed and whether it is being used as a monotherapy or in a combined therapy. The frequency of administration will also be influenced by the above-mentioned factors and particularly the half-life of the inhibitor within the subject being treated.

Optimal dosages to be administered may be determined by those skilled in the art, and will vary with the particular inhibitor in use, the strength of the preparation, and the mode of administration.

Additional factors depending on the particular subject being treated will result in a need to adjust dosages, including subject age, weight, gender, diet, and time of administration.

When the inhibitor is a nucleic acid conventional molecular biology techniques (vector transfer, liposome transfer, ballistic bombardment etc) may be used to deliver the inhibitor to the target tissue.

Known procedures, such as those conventionally employed by the pharmaceutical industry (e.g. in vivo experimentation, clinical trials, etc.), may be used to establish specific formulations for use according to the invention and precise therapeutic regimes (such as daily doses of the gene silencing molecule and the frequency of administration).

Generally, a daily dose of between 0.01 µg/kg of body weight and 0.5 g/kg of body weight of an inhibitor of copine III may be used for the treatment of cancer in the subject, depending upon which specific inhibitor is used. When the inhibitor is an siRNA molecule, the daily dose may be between 1 pg/kg of body weight and 100 mg/kg of body weight, in some embodiments, between approximately 10 pg/kg and 10 mg/kg, or between about 50 pg/kg and 1 mg/kg.

When the inhibitor (e.g. siNA) is delivered to a cell, daily doses may be given as a single administration (e.g. a single daily injection).

Various assays are known in the art to test dsRNA for its ability to mediate RNAi (see for instance Elbashir et al., Methods 26 (2002), 199-213). The effect of the dsRNA according to the present invention on gene expression will typically result in expression of the target gene being inhibited by at least 10%, 33%, 50%, 90%, 95% or 99% when compared to a cell not treated with the RNA molecules according to the present invention.

Similarly, various assays are well-known in the art to test antibodies for their ability to inhibit the biological activity of their specific targets. The effect of the use of an antibody according to the present invention will typically result in biological activity of their specific target being inhibited by at least 10%, 33%, 50%, 90%, 95% or 99% when compared to a control not treated with the antibody.

The term "cancer" refers to a group of diseases in which cells are aggressive (grow and divide without respect to normal limits), invasive (invade and destroy adjacent tissues), and sometimes metastatic (spread to other locations in the body). These three malignant properties of cancers differentiate them from benign tumors, which are self-limited in their growth and don't invade or metastasize (although some benign tumor types are capable of becoming malignant). A particular type of cancer is a cancer forming solid tumours. Such cancer forming solid tumours can be breast cancer, prostate carcinoma or oral squamous carcinoma. Other cancer forming solid tumours for which the methods and inhibitors of the invention would be well suited can be selected from the group consisting of adrenal cortical carcinomas, angiomatoid fibrous histiocytomas (AFH), squamous cell bladder carcinomas, urothelial carcinomas, bone tumours, e.g. adamantinomas, aneurysmal bone cysts, chondroblastomas, chondromas, chondromyxoid fibromas, chondrosarcomas, fibrous dysplasias of the bone, giant cell tumours, osteochondromas or osteosarcomas, breast tumours, e.g. secretory ductal carcinomas, chordomas, clear cell hidradenomas of the skin (CCH), colorectal adenocarcinomas, carcinomas of the gallbladder and extrahepatic bile ducts, combined hepatocellular and cholangiocarcinomas, fibrogenesis imperfecta ossium, pleomorphic salivary gland adenomas head and neck squamous cell carcinomas, chromophobe renal cell carcinomas, clear cell renal cell carcinomas, nephroblastomas (Wilms tumor), papillary renal cell carcinomas, primary renal ASPSCR1-TFE3 t(X;17)(p11;q25) tumors, renal cell carcinomas, laryngeal squamous cell carcinomas, liver adenomas, hepatoblastomas, hepatocellular carcinomas, non-small cell lung carcinomas, small cell lung cancers, malignant melanoma of soft parts, medulloblastomas, meningiomas, neuroblastomas, astrocytic tumours, ependymomas, peripheral nerve sheath tumours, neuroendocrine tumours, e.g. phaeochromocytomas, neurofibromas, oral squamous cell carcinomas, ovarian tumours, e.g. epithelial ovarian tumours, germ cell tumours or sex cord-stromal tumours, pericytomas, pituitary adenomas, posterior uveal melanomas, rhabdoid tumours, skin melanomas, cutaneous benign fibrous histiocytomas, intravenous leiomyomatosis, aggressive angiomyxomas, liposarcomas, myxoid liposarcomas, low grade fibromyxoid sarcomas, soft tissue leiomyosarcomas, biphasic synovial sarcomas, soft tissue chondromas, alveolar soft part sarcomas, clear cell sarcomas, desmoplastic small round cell tumours, elastofibromas, Ewing's tumours, extraskeletal myxoid chondrosarcomas, inflammatory myofibroblastic tumours, lipoblastomas, lipoma, benign lipomatous tumours, liposarcomas, malignant lipomatous tumours, malignant myoepitheliomas, rhabdomyosarcomas, synovial sarcomas, squamous cell cancers, subungual exostosis, germ cell tumours in the testis, spermatocytic seminomas, anaplastic (undifferentiated) carcinomas, oncocytic tumours, papillary carcinomas, carcinomas of the cervix, endometrial carcinomas, leiomyoma as well as vulva and/or vagina tumours. In an embodiment of the invention, the cancer is a cancer of the breast, an ovarian cancer, a stomach cancer, or a uterine cancer, such as uterine serous endometrial carcinoma.

As used herein, the tem "Metastasis" refers to the spread of cancer cells from one organ or body part to another area of the body, i.e. to the formation of metastases. This movement of tumor growth, i.e. metastasis or the formation of metastases, occurs as cancer cells break off the original tumor and spread e.g. by way of the blood or lymph system. Without wishing to be bound by theory, metastasis is an active process and involves an active breaking from the original tumor, for instance by protease digestion of membranes and or cellular matrices, transport to another site of the body, for instance in the blood circulation or in the lymphatic system, and active implantation at said other area of the body.

In one embodiment, the cancer is a copine III-dependent cancer. Copine III-dependent cancers are cancers where copine III has become an essential gene. Copine III-dependent cancers can be easily identified by depleting the cells of copine III expression, and identifying the cancers that are not able to grow, migrate or forming metastases in the absence of it.

The present invention also provides a method of screening compounds to identify those which might be useful for treating cancer in a subject by inhibiting copine III as well as the so-identified compounds.

Copine III, also known as Copine-III, CPN3, KIAA0636 or PRO1071, is a member of the copine family. Copines are a conserved family of ubiquitously expressed proteins found in many eukaryotic organisms (Tomsig and Creutz, 2002). Copines possess two C2 domains (C2D-A and C2D-B) and an A domain; C2Ds are responsible for Ca²+-dependent membrane-binding properties and A domains bind proteins (Tomsig *et al.,* 2003). Copine family members have been shown to bind membranes in various organisms (Church and Lambie, 2003; Gottschalk *et al.,* 2005; Hua *et al.,* 2001). Bioinformatic analysis showed that aspartate residues required for Ca²⁺-binding are conserved in both Copine-III C2Ds. Additionally, between the C2D-B and the A domain of Copine-III there is a Lys-rich cluster, which, in PKCs, has been shown to bind phosphatidylinositol(4,5)P₂ thereby strengthening membrane affinity (Guerrero-Valero *et al.,* 2007). Using IF the present inventors show that Copine-III and ErbB2 co-localize to the PM after HRG stimulation. Moreover, in FRET acceptor photobleaching experiments they measured a FRET efficiency of 8 ± 3 between ErbB2 and Copine-III, which agrees well with the FRET efficiency of 14 ± 3 between ErbB2 receptors (Nagy *et al*., 1998). Taken together, these analysis of Copine-III suggests that it is a Ca²⁺-dependent membrane-binding copine that responds to ErbB2 activation and localizes to the PM in close proximity to the receptor. SEQ ID NO:8 is the amino acid sequence of human copine III.
Copine A domains have been shown to selectivity bind various proteins (Tomsig *et al*., 2003). This suggests that different copines respond to Ca²⁺ signals by transporting selectively bound proteins to phospholipid-containing membranes, where they influence activity of signaling pathways. Using mass spectrometry the present inventors identified seven Copine-III interacting proteins, RACK1 and six other cytoplasmic and nuclear proteins (THO complex subunit 4, Nucleolin, DNA topoisomerase 1, Interleukin enhancer-binding factor 2 and Nuclease-sensitive element-binding protein 1). This is interesting since the present inventors also observed enhanced Copine-III nuclear staining following Ca²⁺ stimulation or HRG treatment and Copine-III nuclear staining was observed on breast and prostate TMAs.

The adaptor protein RACK1, also known as GNB2L1, Guanine nucleotide binding protein (G protein), beta polypeptide 2-like 1, Gnb2-rs1, H12.3, HLC-7, PIG21, Cell proliferation-inducing gene 21 protein, Guanine nucleotide-binding protein subunit beta-like protein 12.3, Human lung cancer oncogene 7 protein, Receptor for Activated C Kinase, Receptor for activated C kinase, Receptor of activated protein kinase C, guanine nucleotide binding protein (G protein), beta polypeptide 2-like 1, lung cancer oncogene 7, proliferation-inducing gene 21, protein homologous to chicken B complex protein, guanine nucleotide binding, acts as a scaffold in numerous signaling events including cell migration, where it has been shown to localize to nascent focal adhesions (FAs) (Cox *et al*., 2003). RACK1 was recently shown to associate with, and be required for FAK activation (Kiely *et al*., 2009). The present invetors provide evidence that Copine-III binds RACK1 and co-localizes with pFAK at focal adhesions in the migrating front of breast cancer cells. Moreover, in Copine-III KD cells they observe decreased Src-activity and decreased FAK-phosphorylation at the Src site (pY925). Phosphorylation of Tyr925FAK by Src is required for proper focal adhesion turnover and cell migration (Brunton *et al*., 2005). A RACK1-Src association has been described (Miller *et al*., 2004). Thus, without wishing to be bound by theory, the present inventors postulate that copine III is a ErbB2- and Ca²⁺-dependent membrane binding protein that functions at focal adhesions, where it contributes to Src activity and tumor cell migration. Since other cellular read-outs, such as proliferation and anchorage-independent growth were unaltered in Copine-III KD T47D cells, Copine-III's influence in response to HRG appears to be specific to migration in these cells.

ErbB2, also known as v-erb-b2 erythroblastic leukemia viral oncogene homolog 2, neuro/glioblastoma derived oncogene homolog (avian), C-erbB-2, CD340, EC 2.7.10.1, HER-2, HER-2/neu, HER2, NEU, NGL, TKR1, erbB-2, herstatin, p185erbB2, CD340 antigen, MLN 19, NEU proto-oncogene, Tyrosine kinase-type cell surface receptor HER2, c-erb B2/neu protein, neuroblastoma/glioblastoma derived oncogene homolog, or v-erb-b2 avian erythroblastic leukemia viral oncogene homolog 2 (neuro/glioblastoma derived oncogene homolog), is a member of the ErbB protein family, more commonly known as the epidermal growth factor receptor family. This protein has no ligand binding domain of its own and therefore cannot bind growth factors. However, it does bind tightly to other ligand-bound EGF receptor family members to form a heterodimer, stabilizing ligand binding and enhancing kinase-mediated activation of downstream signalling pathways, such as those involving mitogen-activated protein kinase and phosphatidylinositol-3 kinase. Allelic variations at amino acid positions 654 and 655 of isoform a (positions 624 and 625 of isoform b) have been reported, with the most common allele, IIe654/IIe655. Amplification and/or overexpression of this gene has been reported in numerous cancers, including breast and ovarian tumors.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

### Examples

### Cell Culture

T47D and SKBr3 breast tumor cells were cultured in Dulbecco's modified Eagle's medium supplemented with 10% fetal bovine serum (FBS) (GIBCO Invitrogen, Basel, Switzerland) and 2mM glutamine at 37 °C, 5% CO₂.

### Mass Spectrometry and Quantification

The Coomassie stained bands were processed by reduction and alkylation of the cysteines followed by tryptic digestion. Peptides were analyzed by nano-HPLC (Agilent 1100 nanoLC system, Agilent Technologies, Santa Clara, USA) coupled to a 4000QTRAP mass spectrometer (Applied Biosystems, Foster City, USA). Peptides were identified searching UniProt data base (version 6.4) restricted to *Homo sapiens* using Mascot (version 2.1, Matrix Science, London, UK)(Perkins *et al*., 1999).

For relative quantification, the required peptide information (e.g. *m*/*z* values of the precursor and fragment ions, retention time) was extracted from the Mascot dta file using "MRM buddy" (unpublished, developed at the FMI, Basel, Switzerland by Alessandro di Cara and Reto Portman). Proteins identified with ≥2 peptides and with a Mascot protein score ≥20 were quantified. For quantification samples were re-injected and analyzed in the multiple reaction monitoring (MRM) mode. Relative quantification was performed by comparing the peak areas that were extracted using Analyst Classic in Analyst 1.4.1.

### Immunofluorescence microscopy

Preparation of cells was performed as described previously (Meira *et al*., 2009). Images were recorded with an Axioskop Zeiss Microscope coupled to a Sony 3 CDD camera. For inhibitor studies, cultures were starved overnight, then preincubated 1.5h with 0.5µM AEE788 (DMSO for controls) and 1 nM HRG (PBS for controls) was added for 20min. Cells were fixed and stained as described (Meira *et al*., 2009).

### Copine-III knockdown

Transient: Copine-III siRNA was obtained from Qiagen (Germantown, MD). Cells were transfected in Optimem medium with the HiPerfect Transfection reagent (Qiagen) according to the protocol, using 2µM siRNA. Successful KD was obtained using the following siRNA sequences: Optimal down-regulation occurred 96h post transfection with the following siRNA sequences: siRNA1 5'-CCAGUUAUCUGUUAAUCAA-3' (SEQ ID NO:2), siRNA2 5'-GGCAAUCUUUCCAAA GUAA-3' (SEQ ID NO:3). LacZ: 5'-GCGGCUGCCGGAAUUUACC-3' (SEQ ID NO:4).

Stable: Bacterial glycerol stocks of pLKO.1-puro vectors containing different shRNA sequences targeting Copine-III were purchased from Sigma (MISSION shRNA library, TCRN_NM_00390S). Infections were done as described in Section III: Lentiviral Infection Protocol from the RNAi Consortium (Cambridge, MA). Copine-III knock-down (KD) was confirmed by western blotting. The shLacZ vector was produced by cloning the 5'-CCGGGCGGCTGCCG GAATTTACCTTCTCGAGGGTAAATTCCGGCAGCCGCTTTTTAATT-3' (SEQ ID NO:5) sequence into the pLKO.1-puro plasmid. Bacterial glycerol stocks of pLKO.1-puro vectors containing different shRNA sequences targeting Copine-III were purchased from Sigma (MISSION shRNA library, TCRN_NM_00390S). Successful knockdown was achieved with: shRNA1: 5'-CCGGCCCTACTGCAATGGAATCCAACTCGAGTTGGATTCCA TTGCAGTAGGGTTTTT-3' (SEQ ID

NO:6), shRNA2: 5'-CCGGGCACAGAAAGGA TTAAGAATTCTCGAGAATTCTTAATCCTTTCTGTGCTTTTT-3' (SEQ ID NO:7).

HEK293 packaging cells were transiently transfected with polyethylenimine (PEI) (Polysciences Inc.) at a PEI:DNA ratio of 4:1. PEI was added to DNA diluted in serum-free media (8µg pLKO.1-puro backbone with shLacZ or shCopine-III, 0.4µg HDM-tat16,0.4µg HDM-HgPM2, 0.4µg pRC-CMV-Rall and 0.8µg HDM-VSV-G), vortexed and incubated at RT for 15min. The PEI/DNA mix was added to the cells and incubated for 16h at 37 °C, at which point the media was changed. Media containing viruses was collected 72h post-transfection, filter sterilized, aliquoted and stored at -80 °C.

T47D and SKBr3 cells were infected by incubation in media containing 8µg/mL polybrene (Sigma) and 600µl of viral supernatant for 24h at 37 °C. The media was then changed and the cells were incubated another 24h at 37 °C. Cells infected with lentivirus for shRNA expression were cultured in medium supplemented with 0.5ug ml⁻¹ puromycin (BD Bioscience, Palo Alto, CA, USA). Successfully infected cells were then maintained using 0.2µg ml⁻¹ puromycin and knock-down of Copine-III was confirmed by western blotting.

### Wound healing assay

Wound healing capability was analyzed using scratch assays. Cells were grown to confluency and starved for 8h in phenol red-free DMEM + 0.1% FBS. Monolayers were scratched and stimulated with 1 nM HRG. Wound healing was monitored using a Widefield TILL5, LONG RUN, Axiovert 200M (5% CO2 and 37°C chamber). Time-lapse images were digitally captured every 20 minutes with a CCD camera over 12 hours. The area recovered by the migrating cells was calculated using ImageJ and plotted in Excel. For IF, cells were stimulated, scratched and allowed to migrate for 1.5h, afterwards cells were treated as described previously (Meira *et al.,* 2009).

### Antibodies and Reagents

Recombinant heregulin β-1 (HRG) was purchased from R&D Systems (Minneapolis, MN, USA) and solubilized in PBS + 0.1% BSA. The following antibodies were used: The serum for the polyconal antibody against a 17- amino acid peptide of Copine-III was provided by Elisabeth A. Grimm (MD Anderson, Houston, TX, USA) (Caudell *et al.,* 2000). The monoclonal Copine-III antibody was generated against the recombinant C2B domain of human Copine-III (AA132 to 278). This antibody was used for all immunofluoresecence (IF) studies. For ErbB2, the 21 N polyclonal antiserum was used in western blot analysis (Hynes *et al.,* 1989). A polyclonal antibody (sc-284) purchased from Santa Cruz (Santa Cruz, CA) was used in IF studies. Other antibodies used in western blot analyses included rabbit anti-GAPDH (ab9385), rabbit anti-Calnexin (ab10286) from Abcam (Cambridge, CA,USA), rabbit anti-p42/44 Erk (#9102), rabbit anti-phosphorylated p42/44 Erk (#9101), rabbit anti-Akt (#9272), rabbit anti-phosphorylated Akt (#9271), rabbit anti-phosphorylated pY925 FAK (#3284) all from Cell Signaling (Danvers, MA, USA), the anti-mouse Src (0-5184) antibody from Upstate, the rabbit anti-phosphorylated Src (44-660G), the rabbit anti-phosphorylated pY861 FAK (07-832) and the rabbit anti-phosphorylated pY397FAK (44-624G) the from Biosource (Invitrogen) and rabbit anti-FAK (sc-932) from Santa Cruz (Santa Cruz Biotechnology Inc., Santa Cruz, USA). The rabbit anti Copine-I antibody (10126-2-AP) was from PTGLabs (Chicago, IL). PTyr was detected with the 4G10 monoclonal antibody (gifted from Juergen Mestan, NIBR, Basel, Switzerland). For the transferrin receptor cells were stained with the Ber-T9 antibody (sc-19675) from Santa Cruz. The following secondary antibodies were used: An alexa488-conjugated goat anti-mouse antibody, an alexa568-conjugated goat anti-rabbit antibody (both Molecular Probes, Eugene, OR, USA) and a Cy3-conjugated donkey anti-rabbit antibody (Jackson ImmunoReasearch Laboratories, Inc., West Grove, PA, USA). DMSO was purchased from Sigma, lonomycin from Calbiochem (Merck, Germany). The small molecule inhibitor AEE788 is a Novartis compound (Basel, Switzerland) (Traxler *et al.,* 2004).

### SILAC

Cells were cultured in medium without arginine (Arg) and lysine (Lys), which was supplemented with a 400-fold excess of ¹²C₆¹⁴N₄ (Arg0) or ¹³C₆¹⁵N₄ (Arg10) and ¹²C₆¹⁴N₂ (Lys0) or ¹³C₆1⁴N₂ (Lys6) (Sigma and Cambridge Isotope Laboratories, Andover, MA, USA). Cells were cultured for at least 5 passages. The level of incorporation was measured by MALDI mass spectrometry (Ultraflex II, Bruker, Bremen, Germany).

### Peptide synthesis and affinity pull-down

Peptides were synthesized as 15-19mers carrying an N-terminal desthiobiotin group followed by a four amino acid linker (Ward *et al.,* 1996) by JPT Peptide Technologies GmbH (Berlin, Germany) in the non-phosphorylated and Tyr-phosphorylated form (sequence: desthiobiotin-SGSGPTAENPEYLGLDVPV-NH₂) (SEQ ID NO:1)

For affinity pull-downs, 2mg Dynabeads MyOne Streptavidin C1 (Invitrogen) were incubated with 150pmol peptide for 30min at room temperature (RT). Heregulin β-1 (HRG) stimulated T47D cells were lysed in 50mM Tris-HCl pH 7.5, 150mM sodium chloride, 1% (v/v) Nonident P-40, protease inhibitors (Complete Tablets, Roche), and 1 mM sodium orthovanadate. 12mg of protein lysate were added and incubated for 4 hours at 4°C, afterwards beads were washed and samples pooled (see Figure 1a). Proteins were eluted in biotin for 30min at 30°C or in SDS buffer at 95°C for 10min, subjected to SDS-polyacrylamide gel electrophoresis (SDS PAGE) and visualized using the Colloidal Blue Staining Kit (Invitrogen).

### Quantification correction and normalization

Ratios (heavy/light) of peak areas for each individual light and heavy form of each peptide were calculated using Excel. Individual peptide ratios were corrected for the purity (given by the manufacturer) and incorporation (measured) of the labeled isotopes. From the corrected and normalized peptide ratios overall protein ratios were determined. Assuming a normal distribution we determined the mean of all protein ratios and set the cut-off for specific binding proteins at 2σ below and above the mean.

### Subcellular fractionation

The cells were washed and detached by scraping in PBS, then homogenized and fractionated following the Membrane Preparation by Homogenization protocol from Current Protocols in Cell Biology online (Unit 7.10, support protocol 1; Wiley Interscience, John Wiley & Sons Inc, Malden, MA). All buffers were supplemented with either 10µM CaCl₂ or 1 mM EGTA. Equal volumes of each fraction were used for subsequent SDS-PAGE and western blotting with specific antibodies.

### Preparation of cell lysates, IP and immunoblotting

For assaying the effect of stimulation the cells were starved in DMEM + 0.1% FBS overnight and subsequently stimulated with 10ng ml⁻¹ HRG for 20 minutes, unless indicated otherwise. Control cells were treated with PBS + 0.1 % BSA for the same time. Whole cell extracts (WCEs) were prepared in lysis buffer (50mM HEPES, pH 7.4, 150mM NaCl, 1% Triton X-100, 1x complete EDTA-free, 1mM sodium orthovanadate) and total protein content was determined using the Bradford Protein Assay (Bio-Rad, Hercules, CA, USA). Immunoprecipitations (IPs) were performed as described previously (Meira *et al.,* 2009)

### FRET acceptor photobleaching

SKBr3 cells were grown on glass coverslips (BD Biosciences, San Diego, CA) coated with 25ug ml⁻¹ rat-tail Collagen I (Roche Diagnostics, Indianapolis, IN) and stimulated 20min with 1nM HRG as described (Meira *et al.,* 2009), except that slides were mounted using Mowiol (Calbiochem, EMD Chemicals, Gibbstown, NJ). Copine-III was visualized using a secondary anti-mouse Alexa488-conjugated antibody. ErbB2 and transferrin receptor were visualized using a secondary anti-rabbit Cy3-conjugated antibody. Acceptor photobleaching was used to assess FRET efficiency on a Zeiss LSM510 confocal microscope equipped with a 63x/1.4 oil immersion objective. Alexa488 was excited with a 488nm laser line, emission measured with a BP505-530 filter. Cy3 was excited with a 543nm laser line, emission measured with a LP560 filter. For each measure Cy3 bleaching had to be over 90% and FRET efficiency (E) was calculated after background subtraction and Alexa488 bleaching correction as follows: E = (I_{D}-I_{DA})/I_{D}, where I_{D} and I_{DA} are Alexa488 intensities in the bleached region after and before photobleaching of Cy3, respectively. For each condition, 4 to 10 cells were assessed.

### Tissue microarray (TMA) design and immunohistochemistry analysis

Cancer specific TMAs were prepared (Kononen et al., 1998) and analyzed as described (Capra *et al.,* 2006; Confalonieri *et al.,* 2009). 2µm sections of the TMA block were cut, mounted on glass slides and processed for immunohistochemistry (IHC). Immunostaining for Copine-III was performed using mouse monoclonal antibody (1:600 dilution) followed by detection with the EnVision Plus/HRP detection system (DAKO, Glostrup, Denmark). A semiquantitative approach was used to evaluate Copine-III expression, which was scored as follows: 0, negative staining; 1, weak; 2, moderate; 3, intense. Samples showing IHC scores ≥1.0 were assigned to the Copine-III-positive expression group (or high expression group), whereas those with IHC scores < 1.0 were considered as Copine-III-negative expression group (or low expression group).

### Results

### Identification of Copine-III as an ErbB2 binding partner by quantitative mass spectrometry

To identify proteins that specifically bind to pTyr1248 of ErbB2, the present inventors used stable isotope labeling of amino acids in cell culture (SILAC) (Blagoev *et al.,* 2003) in combination with an *in-vitro* peptide affinity pull-down approach (Schulze and Mann, 2004), followed by relative quantification by mass spectrometry. Endogenous amino acids in the T47D breast cancer cell line were substituted by their light (Lys0 and Arg0) and heavy (Lys6 and Arg10) forms. Extracts from both populations were subjected to an affinity pull-down using peptides (Tyr and pTyr) encompassing Tyr1248 of the C-terminal tail of ErbB2. Bound proteins were eluted, subjected to SDS-polyacrylamide electrophoresis (PAGE), digested with trypsin and identified by LC-MSMS.

Over 100 proteins were identified in each individual pull-down experiment, however, only proteins identified by ≥2 peptides and with a Mascot protein score ≥20 were selected. For quantification, specific transitions for the light and heavy form of each peptide were measured over time using multiple reaction monitoring (MRM). The ratio of the peak area for the heavy form over that of the light form was calculated. The majority of the proteins yielded a quantification ratio of around 1, reflecting equal binding to the Tyr and the pTyr peptide. A protein was considered as a specific binder if the mean ratio of all its quantified peptides was ≥1.5. Copine-III (acc. AAH07017) was quantified with ratio of 1.8 in three independent experiments.

The selective binding of Copine-III was confirmed using western blot analysis with a Copine-III specific antibody. Tyr1248 and pTyr1248-containing peptides were incubated with T47D cell lysates and used as affinity reagents. Shc, which has been identified as a pTyr1248-binding protein (Dankort *et al.,* 2001; Schulze *et al.,* 2005) was also examined as a control. These results confirmed the specific binding of Copine-III to the pTyr1248-containing peptide.

### Copine-III is a Ca²+-dependent, membrane-binding protein

Copine-III belongs to a family of proteins, which have been shown to be Ca²⁺-dependent, phospholipid binders (Creutz *et al.,* 1998; Tomsig *et al.,* 2003). Copine-III is ubiquitously expressed in human tissues (HUGE data base); however, essentially nothing is known about its role in normal or cancer tissue. It was shown that Copine-III mRNA and protein might be upregulated in response to ErbB2 expression levels and activation (Gharbi *et al.,* 2002; White *et al.,* 2004, Duran et al., 2008). In order to clarify these findings, the present inventors examined Copine-III in more detail using the breast cancer cell lines T47D, with moderate ErbB2 levels, and SKBr3, which has *ERBB2* amplification and very high levels of the receptor (Lane *et al.,* 2000).

The effect of Ca²+on Copine-III's cellular location was first assessed by subcellular fractionation experiments. Cells were lysed in buffer containing CaCl₂ or EGTA and crudely fractionated into cytoplasmic (C) and membrane (M) fractions. In both cell lines Copine-III was enriched in the membrane fraction in the presence of Ca²⁺ and shifted to the GAPDH-containing cytoplasmic fraction after the addition of EGTA . These results demonstrate that Ca²⁺ is required for Copine-III localization to membranes. Calnexin, an integral membrane protein of the endoplasmic reticulum (ER), was detected in the crude membrane fraction. Thus, the present inventors used immunofluorescence microscopy (IF) to see whether Copine-III bound to the ER or to the plasma membrane (PM) in response to Ca²⁺, by treating the cells with the Ca²⁺-ionophore ionomycin. Copine-III was detected using a monoclonal antibody directed against its C2B domain. The specificity of the antibody was verified using Copine-III knock-down (KD) cells. In control DMSO treated cells, Copine-III was found mainly in the cytoplasm; treatment with ionomycin caused its re-localization to the PM. In T47D cells, Copine-III staining was enriched at cell-cell adhesion membranes, while in SKBr3 cells Copine-III localized to specific regions at the PM. Thus, Copine-III is a Ca²⁺-dependent membrane-binding protein.

### Copine-III and ErbB2 co-Iocalize and interact at the PM

The present inventors explored the localization of Copine-III and ErbB2 in the breast cancer cell lines using IF. In control T47D cells, Copine-III was diffusely distributed throughout the cytoplasm. In response to HRG, Copine-III re-localized to the PM, where it co-localized with activated ErbB2. In control SKBr3 cells, most of Copine-III was diffusely distributed throughout the cytoplasm, however, a portion also co-localized with the receptor at the PM. In SKBr3 cells ErbB2 has high levels of pTyr, even in the absence of ligands. HRG treatment caused ErbB2 to distribute along the PM, and increased Copine-III at the membrane, where it co-localized with ErbB2.

Next the present inventors used fluorescence resonance electron transfer (FRET) acceptor photobleaching (Kenworthy, 2001) to examine the interaction of Copine-III and ErbB2 in fixed, HRG treated SKBR3 cells. Copine-III and ErbB2 were labeled with specific primary antibodies and with a donor (Alexa488) and acceptor (Cy3) fluorophore-conjugated secondary antibody, respectively. Acceptor photobleaching was performed and FRET efficiency was measured at three regions: (1) at the membrane where both proteins co-localize, (2) in the cytoplasm where there is no co-localization and (3) outside the cell to measure background fluorescence. FRET efficiency (E) was calculated by comparing the Alexa488 intensities in the bleached region after and before photobleaching of Cy3, respectively. FRET efficiency was measured between ErbB2 and transferrin receptor (TR) and ErbB2 and Copine-III. ErbB2 co-localized with both TR and Copine-III at the PM, however, no FRET efficiency was measured between ErbB2 and TR (E = 0.6 ± 1), showing that these receptors co-localize but do not interact. Importantly, a significant FRET efficiency was found for ErbB2 and Copine-III (E = 8.3 ± 1.3). As a control, no FRET efficiency was measured for only Alexa488 labeling of Copine-III. These results suggest that ErbB2 and Copine-III interact at the PM in HRG-stimulated SKBr3 cells.

### Copine-III interacts with the active ErbB2 receptor at the PM

To test directly if Copine-III interacts with active ErbB2, SKBr3 cells were pretreated with the ErbB tyrosine kinase inhibitor (TKI) AEE788 (Traxler *et al.,* 2004) before stimulating with HRG. In TKI-treated cells there was a dramatic decrease in the pTyr content of ErbB2 as shown by immunoprecipitating the receptor and probing the immunoprecipitates (IP) with a pTyr specific antibody. Additionally, the activity of the Akt and Erk pathways were also reduced. IF revealed that the intense regions of ErbB2-membrane staining in control cells were lost in TKI-treated cells, and the receptor re-localized to specific intracellular regions. Strikingly, in AEE788 treated cells there was little or no Copine-III at the PM in comparison to control cells, where there was intense membrane staining. Taken together, these results show that Copine-III requires active ErbB2 signaling at the PM for its localization there.

The present inventors examined complexes of Copine-III and ErbB2 using co-IP experiments. ErbB2 was found in IPs of Copine-III from lysates of T47D and SKBr3 cells. In control cells an association of ErbB2 and Copine-III was detected, reflecting autocrine receptor activity in T47D cells (Meira et al., 2009) and ErbB2 overexpression in SKBr3 cells (Lane *et al.,* 2000). In Copine-III IPs from lysates of HRG-treated cells there was an increase in complexed, active pTyr-containing ErbB2. Copine-III does not possess an SH2- or a PTB-pTyr binding domain. Shc has both domains and has also been found associated with pTyr1248 (Dankort *et al.,* 1997; Schulze *et al.,* 2005). Shc was detected in Copine-III IPs from lysates of T47D cells, and Shc levels increased in response to HRG. Together, these data provide evidence that Copine-III is found in complexes with ErbB2 and with Shc, the latter perhaps mediating Copine-III binding to the receptor.

### Copine-III interacts with RACK1 and both proteins complex with ErbB2

To gain more insight into the role of Copine-III in ErbB2-mediated cellular signaling, the present inventors sought to identify Copine-III binding partners. For this, SKBr3 cells with incorporated light or heavy isotopes of lysine and arginine (SILAC) were transfected with an empty control vector or a Flagtagged Copine-III, respectively. After stimulation with HRG, anti-Flag affinity purification was performed and Copine-III binding partners were identified by MRM. The analysis yielded several putative binding partners, one of these being RACK1, receptor of activated C kinase (Ron *et al.,* 1994). In an independent experiment the present inventors quantified the relative amounts of RACK1 in Copine-III IPs from SKBr3 lysates by MRM. They found a 1.5-fold enrichment of RACK1 in Copine-III IPs vs. control IPs, confirming the interaction. Furthermore, an examination of ErbB2 IPs from SKBr3 and T47D cells revealed complexed Copine-III, Shc and RACK1.

### Copine-III localizes to focal adhesions and is required for ErbB2-dependent tumor cell migration

The adaptor protein RACK1 acts as a scaffold in numerous signaling events including cell migration, where it has been shown to localize to nascent focal adhesions (FAs) (Cox *et al.,* 2003). The present inventor's finding that Copine-III interacts with RACK1 prompted them to examine whether Copine-III might also have a role in cell migration. First, they used IF to examine Copine-III localization. Copine-III and phosphorylated focal adhesion kinase (pTyr397FAK) were both diffusely present in the cytoplasm of control T47D cells and upon HRG treatment co-localized at the PM, suggesting that Copine-III is a FA-associated protein.

Next they examined the role of Copine-III in cell migration using a KD approach. Two pools of Copine-III KD cells and a pool expressing the control (LacZ) vector were generated in T47D cells. Copine-III KD was almost complete in pool 2, while it was approximately 50% in pool 1. The effect of Copine-III KD on migratory ability of T47D cells was examined using a wound healing assay. Wound closure of control and Copine-III KD cultures left untreated or stimulated with HRG was monitored over 12h. Basal migration of control cells was low and not affected by Copine-III KD. Importantly, compared to control cells, HRG-stimulated Copine-III KD cells were significantly impaired in their ability to migrate into the wound (p<0.005).

The localization of Copine-III, pTyr397FAK and ErbB2 was also examined in wounded monolayers. In untreated cells none of these proteins strongly localized to the cell membrane. In contrast, in HRG-treated cultures, Copine-III, pTyr397FAK and ErbB2 were each present in membrane protrusions at the edge of the wounded area. Moreover, a high degree of overlap between Copine-III and pTyr397FAK staining and Copine-III and ErbB2 staining was evident. In Copine-III KD cells, pTyr397FAK and ErbB2 were localized to membrane protrusions in response to HRG, showing that these events are not dependent upon Copine-III.

Src and FAK, two proteins with important roles in cell migration were further analyzed. A western analysis revealed that Src activity increased in control HRG treated cells, as shown by the increase in pTyr418Src levels. In contrast, there was little or no increase in pTyr418Src levels in the two Copine-III KD pools. Moreover, the level of pTyr925FAK, a site that has been shown to require Src kinase activity to become phosphorylated (Brunton *et al.,* 2005), also increased in HRG-treated control cultures, while little or no increase was observed in Copine-III KD pools, attesting to the decrease in Src activity. In contrast to the effects on Src kinase activity, activation of the Akt and Erk pathways was similar in response to HRG-treatment in the Copine-III KD cultures and the control cells. These findings imply that Copine-III might be a component of focal adhesions that is required for Src activity and thus for regulating focal adhesion turnover and ErbB2-mediated migration in response to HRG.

### Copine-III expression in human cancer

The present inventors examined Copine-III and ErbB2 expression levels in breast tumor cell lines and primary tumors. Copine-III was expressed to varying degrees in the breast cancer cells lines, with the highest levels in SKBr3 and BT474, both having *ERBB2* amplification. These data correlate with previous publications showing Copine-III upregulation in response to ErbB2 overexpression (Gharbi *et al*., 2002; White *et al.,* 2004). The present inventors also examined Copine-III RNA expression in 49 primary breast tumors (Farmer *et al.,* 2005). Plotting log gene expression values of *ERBB2* against expression values for *CPNE3* revealed that high levels of *CPNE3* were significantly correlated with *ERBB2* amplification; 10 of 11 tumors with the highest level of Copine-III also possessed the amplicon. The present inventors also performed an analysis of Copine-III expression in different human tumors, initially examining data from published transcriptome studies (www.oncomine.org) (Rhodes *et al.,* 2004). They found that *CPNE3* expression increased significantly in metastatic prostate cancer in comparison to normal prostate and non-metastatic tumors [data from (Yu *et al.,* 2004)]. Moreover, in comparison to normal ovary, there were significantly higher levels of *CPNE3* in ovarian endometrioid adenocarcinoma (OEA), the second most common type of ovarian cancer [data from (Hendrix *et al.,* 2006)].

Based on these findings the present inventors performed IHC staining of Copine-III in breast, prostate and ovarian TMAs. There was a tendency for Copine-III to be up-regulated in the epithelial component of cancer tissue compared to normal tissue. Some Copine-III staining was evident in normal breast, while normal prostate and ovarian tissues have very low levels of Copine-III. Tumors of all three types showed higher Copine-III levels. These finding suggest that the up-regulation trend revealed by the Oncomine analysis is retained at the protein level, implying a potential biological function of Copine-III in human cancer.

ErbB2 has been shown to play a pivotal role in various types of human cancer. In breast cancer, ErbB2 overexpression correlates with aggressive disease and a metastatic phenotype (Hynes and Lane, 2005). In search of novel ErbB2 effector proteins, the present inventors thus identified Copine-III via its ability to specifically bind an ErbB2 pTyr1248-containing peptide. Our results show that Copine-III interacts with ErbB2 at the plasma membrane of breast tumor cells in response to HRG treatment. Moreover, they show that Copine-III binds the focal adhesion-associated protein RACK1 and is also present in focal adhesions in cells with active ErbB2. Importantly, KD of Copine-III in T47D breast tumor cells causes a decrease in HRG-induced migration. These results thus indicate that Copine-III is a novel ErbB2-interacting protein that link ErbB2 to focal adhesions, thereby contributing to HRG induced cell motility.

As mentioned herein above, there was some contradicting evidence in primary human tumors for a functional relationship between Copine-III and ErbB2, since Copine-III mRNA and protein levels were shown to soemtimes correlate with ErbB2 expression (Gharbi *et al.,* 2002; White *et al.,* 2004; Duran *et al*., 2008). Despite these contradictions, the present inventors have extended these studies by examining a panel of 49 breast tumors (Farmer *et al.,* 2005) and showing that, surprisingly, there is a significant correlation between *CPNE3* RNA levels, and *ERBB2* gene amplification and expression,. Furthermore, data mining (Oncomine) and data from inventors' own TMAs provide evidence that Copine-III levels are increased in prostate and ovarian tumors. Taken together, these results indicate that Copine-III function might be important in multiple cancer types. Moreover, the present results implicate Copine-III as an important player in ErbB2-mediated cancer cell motility in ErbB2-mediated cancers.

### References

Akiyama T, Matsuda S, Namba Y, Saito T, Toyoshima K, Yamamoto T (1991). The transforming potential of the c-erbB-2 protein is regulated by its autophosphorylation at the carboxyl-terminal domain. Mol Cell Biol 11: 833-42.
Benes CH, Wu N, Elia AE, Dharia T, Cantley LC, Soltoff SP (2005). The C2 domain of PKCdelta is a phosphotyrosine binding domain. Cell 121: 271-80.
Blagoev B, Kratchmarova I, Ong SE, Nielsen M, Foster LJ, Mann M (2003). A proteomics strategy to elucidate functional protein-protein interactions applied to EGF signaling. Nat Biotechnol 21: 315-8.
Boulay A, Breuleux M, Stephan C, Fux C, Brisken C, Fiche M et al (2008). The Ret receptor tyrosine kinase pathway functionally interacts with the ERalpha pathway in breast cancer. Cancer Res 68: 3743-51.
Brunton VG, Avizienyte E, Fincham VJ, Serrels B, Metcalf CA, 3rd, Sawyer TK et al (2005). Identification of Src-specific phosphorylation site on focal adhesion kinase: dissection of the role of Src SH2 and catalytic functions and their consequences for tumor cell behavior. Cancer Res 65: 1335-42.
Capra M, Nuciforo PG, Confalonieri S, Quarto M, Bianchi M, Nebuloni M et al (2006). Frequent alterations in the expression of serine/threonine kinases in human cancers. Cancer Res 66: 8147-54.
Caudell EG, Caudell JJ, Tang CH, Yu TK, Frederick MJ, Grimm EA (2000). Characterization of human copine III as a phosphoprotein with associated kinase activity. Biochemistry 39: 13034-43.
Confalonieri S, Quarto M, Goisis G, Nuciforo P, Donzelli M, Jodice G et al (2009). Alterations of ubiquitin ligases in human cancer and their association with the natural history of the tumor. Oncogene*.*
Church DL, Lambie EJ (2003). The promotion of gonadal cell divisions by the Caenorhabditis elegans TRPM cation channel GON-2 is antagonized by GEM-4 copine. Genetics 165: 563-74.
Citri A, Yarden Y (2006). EGF-ERBB signalling: towards the systems level. Nat Rev Mol Cell Biol 7: 505-16.
Cox EA, Bennin D, Doan AT, O'Toole T, Huttenlocher A (2003). RACK1 regulates integrin-mediated adhesion, protrusion, and chemotactic cell migration via its Src-binding site. Mol Biol Cell 14: 658-69.
Creutz CE, Tomsig JL, Snyder SL, Gautier MC, Skouri F, Beisson J et al (1998). The copines, a novel class of C2 domain-containing, calcium-dependent, phospholipid-binding proteins conserved from Paramecium to humans. J Biol Chem 273: 1393-402.
Dankort D, Maslikowski B, Warner N, Kanno N, Kim H, Wang Z et al (2001). Grb2 and Shc adapter proteins play distinct roles in Neu (ErbB-2)-induced mammary tumorigenesis: implications for human breast cancer. Mol Cell Biol 21: 1540-51.
Dankort DL, Wang Z, Blackmore V, Moran MF, Muller WJ (1997). Distinct tyrosine autophosphorylation sites negatively and positively modulate neu-mediated transformation. Mol Cell Biol 17: 5410-25.
Duran MC, Vega F, Moreno-Bueno G, Artiga MJ, Sanchez L, Palacios J, Ridley A, Timms JF (2008). Characterisation of tumoral markers correlated with ErbB2 (HER2/Neu) overexpression and metastasis in breast cancer. Proteomics Clin. Appl. 2:1313-26.
Farmer P, Bonnefoi H, Becette V, Tubiana-Hulin M, Fumoleau P, Larsimont D et al (2005). Identification of molecular apocrine breast tumours by microarray analysis. Oncogene 24: 4660-71.
Gharbi S, Gaffney P, Yang A, Zvelebil MJ, Cramer R, Waterfield MD et al (2002). Evaluation of two-dimensional differential gel electrophoresis for proteomic expression analysis of a model breast cancer cell system. Mol Cell Proteomics 1: 91-8.
Gottschalk A, Almedom RB, Schedletzky T, Anderson SD, Yates JR, 3rd, Schafer WR (2005). Identification and characterization of novel nicotinic receptor-associated proteins in Caenorhabditis elegans. Embo J 24: 2566-78.
Graus-Porta D, Beerli RR, Daly JM, Hynes NE (1997). ErbB-2, the preferred heterodimerization partner of all ErbB receptors, is a mediator of lateral signaling. Embo J 16: 1647-55.
Guerrero-Valero M, Marin-Vicente C, Gomez-Fernandez JC, Corbalan-Garcia S (2007). The C2 domains of classical PKCs are specific Ptdlns(4,5)P2-sensing domains with different affinities for membrane binding. J Mol Biol 371: 608-21.
Hazan R, Margolis B, Dombalagian M, Ullrich A, Zilberstein A, Schlessinger J (1990). Identification of autophosphorylation sites of HER2/neu. Cell Growth Differ 1: 3-7.
Hendrix ND, Wu R, Kuick R, Schwartz DR, Fearon ER, Cho KR (2006). Fibroblast growth factor 9 has oncogenic activity and is a downstream target of Wnt signaling in ovarian endometrioid adenocarcinomas. Cancer Res 66: 1354-62.
Holbro T, Beerli RR, Maurer F, Koziczak M, Barbas CF, 3rd, Hynes NE (2003). The ErbB2/ErbB3 heterodimer functions as an oncogenic unit: ErbB2 requires ErbB3 to drive breast tumor cell proliferation. Proc Natl Acad Sci U S A 100: 8933-8.
Holbro T, Hynes NE (2004). ErbB receptors: directing key signaling networks throughout life. Annu Rev Pharmacol Toxicol 44: 195-217.
Hua J, Grisafi P, Cheng SH, Fink GR (2001). Plant growth homeostasis is controlled by the Arabidopsis BON1 and BAP1 genes. Genes Dev 15: 2263-72.
Hynes NE, Lane HA (2005). ERBB receptors and cancer: the complexity of targeted inhibitors. Nat Rev Cancer 5: 341-54.
Hynes NE, Gerber HA, Saurer S, Groner B (1989). Overexpression of the c-erbB-2 protein in human breast tumor cell lines. J Cell Biochem 39: 167-73.
Keely PJ, Westwick JK, Whitehead IP, Der CJ, Parise LV (1997). Cdc42 and Rac1 induce integrin-mediated cell motility and invasiveness through PI(3)K. Nature 390: 632-6.
Kenworthy AK (2001). Imaging protein-protein interactions using fluorescence resonance energy transfer microscopy. Methods 24: 289-96.
Kheifets V, Mochly-Rosen D (2007). Insight into intra- and inter-molecular interactions of PKC: design of specific modulators of kinase function. Pharmacol Res 55: 467-76.
Kiely PA, Baillie GS, Barrett R, Buckley DA, Adams DR, Houslay MD et al (2009). Phosphorylation of RACK1 on tyrosine 52 by c-Abl is required for IGF-I-mediated regulation of focal adhesion kinase (FAK). J Biol Chem.
Klemke RL, Cai S, Giannini AL, Gallagher PJ, de Lanerolle P, Cheresh DA (1997). Regulation of cell motility by mitogen-activated protein kinase. J Cell Biol 137: 481-92.
Lane HA, Beuvink I, Motoyama AB, Daly JM, Neve RM, Hynes NE (2000). ErbB2 potentiates breast tumor proliferation through modulation of p27(Kip1)-Cdk2 complex formation: receptor overexpression does not determine growth dependency. Mol Cell Biol 20: 3210-23.
Marone R, Hess D, Dankort D, Muller WJ, Hynes NE, Badache A (2004). Memo mediates ErbB2-driven cell motility. Nat Cell Biol 6: 515-22.
McCahill A, Warwicker J, Bolger GB, Houslay MD, Yarwood SJ (2002). The RACK1 scaffold protein: a dynamic cog in cell response mechanisms. Mol Pharmacol 62: 1261-73.
Meira M, Masson R, Stagljar I, Lienhard S, Maurer F, Boulay A et al (2009). Memo is a cofilin-interacting protein that influences PLCgamma1 and cofilin activities, and is essential for maintaining directionality during ErbB2-induced tumor-cell migration. J Cell Sci 122: 787-97.
Miller LD, Lee KC, Mochly-Rosen D, Cartwright CA (2004). RACK1 regulates Src-mediated Sam68 and p190RhoGAP signaling. Oncogene 23: 5682-6.
Nagy P, Bene L, Balazs M, Hyun WC, Lockett SJ, Chiang NY et al (1998). EGF-induced redistribution of erbB2 on breast tumor cells: flow and image cytometric energy transfer measurements. Cytometry 32: 120-31.
Nalefski EA, Falke JJ (1996). The C2 domain calcium-binding motif: structural and functional diversity. Protein Sci 5: 2375-90. Patterson RL, van Rossum DB, Nikolaidis N, Gill DL, Snyder SH (2005). Phospholipase C-gamma: diverse roles in receptor-mediated calcium signaling. Trends Biochem Sci 30: 688-97.
Perkins DN, Pappin DJ, Creasy DM, Cottrell JS (1999). Probability-based protein identification by searching sequence databases using mass spectrometry data. Electrophoresis 20: 3551-67.
Rhodes DR, Yu J, Shanker K, Deshpande N, Varambally R, Ghosh D et al (2004). ONCOMINE: a cancer microarray database and integrated data-mining platform. Neoplasia 6: 1-6.
Ron D, Chen CH, Caldwell J, Jamieson L, Orr E, Mochly-Rosen D (1994). Cloning of an intracellular receptor for protein kinase C: a homolog of the beta subunit of G proteins. Proc Natl Acad Sci U S A 91: 839-43.
Ross JS, Fletcher JA (1998). The HER-2/neu oncogene in breast cancer: prognostic factor, predictive factor, and target for therapy. Stem Cells 16: 413-28.
Schlessinger J (2000). Cell signaling by receptor tyrosine kinases. Cell 103: 211-25.
Schulze WX, Deng L, Mann M (2005). Phosphotyrosine interactome of the ErbB-receptor kinase family. Molecular Systems Biology*.*
Schulze WX, Mann M (2004). A novel proteomic screen for peptide-protein interactions. J Biol Chem 279: 10756-64.
Slamon DJ, Clark GM, Wong SG, Levin WJ, Ullrich A, McGuire WL (1987). Human breast cancer: correlation of relapse and survival with amplification of the HER-2/neu oncogene. Science 235: 177-82.
Traxler P, Allegrini PR, Brandt R, Brueggen J, Cozens R, Fabbro D et al (2004). AEE788: a dual family epidermal growth factor receptor/ErbB2 and vascular endothelial growth factor receptor tyrosine kinase inhibitor with antitumor and antiangiogenic activity. Cancer Res 64: 4931-41.
Tomsig JL, Creutz CE (2002). Copines: a ubiquitous family of Ca(2+)-dependent phospholipid-binding proteins. Cell Mol Life Sci 59: 1467-77.
Tomsig JL, Snyder SL, Creutz CE (2003). Identification of targets for calcium signaling through the copine family of proteins. Characterization of a coiled-coil copine-binding motif. J Biol Chem 278: 10048-54.
Tomsig JL, Sohma H, Creutz CE (2004). Calcium-dependent regulation of tumour necrosis factor-alpha receptor signalling by copine. Biochem J 378: 1089-94.
Traxler P, Allegrini PR, Brandt R, Brueggen J, Cozens R, Fabbro D et al (2004). AEE788: a dual family epidermal growth factor receptor/ErbB2 and vascular endothelial growth factor receptor tyrosine kinase inhibitor with antitumor and antiangiogenic activity. Cancer Res 64: 4931-41.
Ward CW, Gough KH, Rashke M, Wan SS, Tribbick G, Wang J (1996). Systematic mapping of potential binding sites for Shc and Grb2 SH2 domains on insulin receptor substrate-1 and the receptors for insulin, epidermal growth factor, platelet-derived growth factor, and fibroblast growth factor. J Biol Chem 271: 5603-9.
White SL, Gharbi S, Bertani MF, Chan HL, Waterfield MD, Timms JF (2004). Cellular responses to ErbB-2 overexpression in human mammary luminal epithelial cells: comparison of mRNA and protein expression. Br J Cancer 90: 173-81.
Yarden Y, Sliwkowski MX (2001). Untangling the ErbB signalling network. Nat Rev Mol Cell Biol 2: 127-37.
Yu YP, Landsittel D, Jing L, Nelson J, Ren B, Liu L et al (2004). Gene expression alterations in prostate cancer predicting tumor aggression and preceding development of malignancy. J Clin Oncol 22: 2790-9.

## Claims

1. A method for treating cancer in a subject by modulating copine III by administering to said subject a therapeutically effective amount of a modulator of copine III.

2. The method of claim 1 wherein copine III is modulated by an inhibitor.

3. The method of claims 1 or 2 wherein the inhibitor is an antibody.

4. The method of claims 1 or 2 wherein the inhibitor decreases or silences the expression of copine III.

5. The method of claim 4 wherein the inhibitor is a siRNA.

6. The method of any of claims 1 to 5 wherein the subject is a mammal, for instance a human subject.

7. The method of any of claims 1 to 6 wherein the ErbB2-pathway is misregulated in the cells of the cancer, for instance wherein the *ErbB2* gene is amplified or overexpressed in the cancer cells.

8. The method of any of claims 1 to 7 wherein the cancer is a cancer of the breast, an ovarian cancer, a stomach cancer, or a uterine cancer, such as uterine serous endometrial carcinoma.

9. The method of any of claims 1 to 8 wherein the modulation of copine III reduces metastasis formation.

10. A siRNA decreasing or silencing copine III, for use as a medicament to treat cancer.

11. An antibody specifically binding to copine III, for use as a medicament to treat cancer.

12. The antibody of claim 9, wherein said antibody inhibits the binding of copine III to its ErbB2 or to RACK1.

13. The siRNA of claim 10 or the antibody of claim 11 or 12, wherein the ErbB2-pathway is misregulated in the cells of the cancer, for instance wherein the *ErbB2* gene is overexpressed or amplified in the cancer cells, or wherein cancer is a cancer of the breast, an ovarian cancer, a stomach cancer, or a uterine cancer, such as uterine serous endometrial carcinoma.

14. A method for the identification of a substance that modulates the expression of copine III and/or biological activity, which method comprises:
(i) contacting a copine III polypeptide or a fragment thereof having the biological activity of copine III, a polynucleotide encoding such a polypeptide or polypeptide fragment, an expression vector comprising such a polynucleotide or a cell comprising such an expression vector, and a test substance under conditions that in the absence of the test substance would permit copine III expression and/or biological activity; and
(ii) determining the amount of copine III expression and/or biological activity, to determine whether the test substance modulates copine III biological activity and/or expression, wherein a test substance which modulates copine III biological activity and/or expression is a potential therapeutical agent to treat cancer.

15. A method of diagnosing cancer comprising the step of assessing the level of expression of copine III in a sample from a subject.
